# EUROPEAN PATENT APPLICATION

(11) **EP 1 587 017 A2**
(43) Date of publication of application: **19.10.2005**
(21) Application number: 05102976.7
(22) Date of filing: 14.04.2005
(51) Int. Cl.: G06F 19/00

(54) **Data management system**

(30) Priority: 15.04.2004 EP 04090144
(71) Applicant: Biotronik GmbH & Co. KG, 12359 Berlin (DE)
(72) Inventor: DIEM, Björn Henrik, 12161, Berlin (DE); PAULE, Stefan, 96117, Drosendorf (DE); PETSCH, Norbert, 91054, Erlangen (DE); URBASZEK, Albrecht, 91353, Hausen (DE)
(74) Representative: Eisenführ, Speiser & Partner

(57) **Abstract**

The invention is concerned with a data management system for processing of physiological or technical data to generate a display control signal. The signal comprises a patient data interface to receive data, from physiological parameter of a patient or technical parameter of an implant like a cardiac pacemaker and a user interface to receive data to be entered by a user. A data base is adapted to store data received via the patient data interface and to retrieve stored data. A processing unit is connected to the user interface, and is adapted to retrieve and to process data stored in the data base and to create a display control signal for a graphical data display. The processing unit is connected to a rule base which comprises rules defining alarm states. The processing unit is adapted to create a display control signal for displaying an alarm symbol in case of an alarm state.

## Description

The invention relates to a data management system for processing of physiological or technical data or both to generate a display control signal. The data management system comprises a data interface have a patient data interface and a user interface. The patient data interface is adapted to receive data which represent physiological parameters of a patient or technical parameters of a medical device, in particular of an implant like a cardiac pacemaker or a defibrillator, cardioverter or the like. The user interface is adapted to receive data to be entered by a user. The user interface may be a keyboard or a touch screen or a voice interface.

The data management further comprises a data base adapted to store data received via the patient data interface and to retrieve stored data. Furthermore, the data management system comprises a processing unit which is at least indirectly connected to the user interface and to a rule base. Said processing unit is adapted to retrieve and to process data stored in the data base according to rules which are stored in the rule base, thereby creating a display control signal for a graphical data display such that the display control signal depends on said data and said rules.

Such data management system in general is already known and serves for the purpose to remotely display data gathered for example by an implant, see for example WO 2004/000111, WO 03/090150, WO 03/088830, WO 03/077752, WO 02/082984, WO 02107816 and WO 02/02004. Such data management system is used in a so called home monitoring environment wherein multiple implants are linked to a central service center via wireless or landline telemetry. Data received from the implants is stored in a central data base and can be retrieved for example by a physician to determine the latest health states of a patient or operational states of the implant.

Data gathered by the implants can be technical data relating to the implant itself, for example the states of the pacing leads of a pacemaker or the states of a pacemaker's battery. The data collected by the implant as well include physical data related to the patient like a natural heart rate of the patient, events of tachycardia or fibrillation or all kind of other data which can be collected automatically by an implantable medical device (IMD).

An advantage of such a home monitoring environment is that a physician has central access to a large number of data allowing all kind of comparisons or calculations or statistics. On the other hand, physicians are many times facing an information overload.

It is an object of the invention to provide for an improved data management system.

According to the invention, the object is achieved by a data management system according to claim 1 wherein said processing unit is connected to a rule base and adapted to process the data stored in the data base according to the rules stored in the rule base. It has proven to be advantageous, if the data base for collected data and a rule base defining how the data has to be processed are separate from each other. This is to be understood, that a rule base is not a program in the usual sense even though a program also defines rules as how to process data. A rule base comprises a set of rules. A program like an expert system accesses these rules in order to process data stored in the data base. In a sense a rule base is a kind of a meta program. The advantage of a rule base is that the rules are easily accessible and easily manageable by a physician without programming skills.

The rules stored in the rule base comprise rules defining alarm states in a way that an alarm state is to be determined by the processing unit, if the data stored in the data base indicate an acute health disaster of a patient or an acute failure of a device. The processing unit is adapted to create a display control signal for displaying an alarm symbol depending on a user identification signal received from the user interface such that for each patient or device being assigned to an identified user all actual alarm states are immediately displayed by an alarm symbol.

When using such data management system, a physician first has to identify himself for example by logging in into a computer, thereby creating an identification signal. Then, all actual alarm states are displayed to the physician. This avoids the need for a physician to manually determine whether any of the patient or devices to be cared for by the physician is in a critical state. In addition, a physician himself or any other expert can set up the rules which lead to the display of an alarm state so that the rules a physician would apply to determine a critical state manually can be entered beforehand in order to automatically create alarm symbols displaying all actual alarm states.

In a preferred embodiment the rule base comprises rules which define warning states such that a warning state is determined by the processing unit if said data indicates an upcoming health complication or a minor technical failure. In such preferred embodiment the processing unit is adapted to generate a display control signal displaying for each patient or device being assigned to an identified user a warning symbol on a display, if for said patient or device a warning state as defined by the rule base is given by an actual set of data for said patient or device. A warning state is defined by data, which indicate a less hazardous health complication or technical failure compared to an alarm state. The rules defining a warning state are also configurable by a physician or another expert. Having two different kinds of symbols for different degrees of health complications further improves the overview a physician gains by using the inventive data management system. For example, alarm states may be indicated by a red alarm symbol, whereas warning states may be indicated by a yellow or orange warning symbol.

A further improvement of the data management system comprises a processing unit which is adapted to generate a display control signal to display an ok-symbol for each person or device for which neither a warning state nor an alarm state is given.

Such an ok-symbol may be of green color in order to easily discriminate the ok-symbol from an alarm symbol or a warning symbol. To discriminate between three states, alarm state, warning state and ok-state, has proven to be very useful in order to provide for an easy access to the information stored in the data base without oversimplifying the information.

The processing unit is preferably adapted to receive a user identification signal via the user interface and to immediately generate a display control signal which initiates immediate display of all actual alarm symbols given by an actual set of data being assigned to patients or devices associated to the user identified by the identification signal. A further improved processing unit is adapted to also immediately display all actual warning states. Displaying all alarm states and, in a preferred embodiment, all actual warning states as well immediately after a login of a physician draws the physicians attention to each patient or device suffering from a major problem in as short a time as possible.

Furthermore, providing for the possibility not only to configure the rule base in order to define certain warning states, the physician shall have the possibility to select all or only a part of the warning states defined by the rules in the rule base for immediate display after login. Providing for such selection of warnings states to be immediately displayed by a warning symbol allows a physician to select those warning states he is interested in, in particular if the rules and the rule base are defined by another expert.

In a preferred mode, the processing unit is adapted to generate a display control signal immediately after login of a physician such that the display control signal causes a list of all names of all patients or devices assigned to that physician and either an alarm symbol, or a warning symbol or an ok-symbol for each listed patient or device. Thus, the physician is provided with a comprehensive overview of the states of all of the patients or devices associated to that physician.

Preferably, in such list those patients or devices for which an alarm state is given are listed atop together with an alarm symbol.

In a preferred embodiment of the management system the data interface comprises a select interface, which is connected to the processing unit. The processing unit is adapted to retrieve further information assigned to a patient or device in response to a first type of a select signal received via the select interface and to generate a display control signal to display said further information. Said further information preferably comprises information to a set of categories characterizing a general status of the selected patient or the selected device. In a further preferred embodiment a second type of a select signal is optionally provided to select one of the categories displayed thereby initiating a display of the actual data which are stored in the data base. This data management system allows easy three-level retrieval of the data stored in the data base. It is an advantage of the preferred embodiment that the data stored in the data base itself can be viewed by a physician.

The object of the invention is also achieved by a data storage means which comprises a program for the forming of a graphical display of physiological or technical data or both. The program comprises the steps of retrieval of data from a data base, processing of the data and generating a display control signal dependent on such processing of the data.

Preferably, the step of processing said retrieved data comprises the sub steps of accessing a rule base, applying rules stored in the rule base on said retrieved data, determining alarm states defined by said rules and generating a display control signal displaying an alarm symbol if an alarm state is determined.

A further improvement of the data management system comprises a communication unit which is adapted to generate a notification signal in case of a critical situation as detected by the processing unit. Based on this notification signal, a notification message is sent to the physician who is assigned to the patient showing the critical state via a configurable communication pathway, e.g., E-Mail, Fax, SMS or Pager. For this purpose, the communication unit comprises a notification message configuration database comprising physician identification data elements which identify the physician to be notified by a respective E-mail address or a Fax number or a mobile phone number.

In a preferred embodiment, the notification message may contain a selection of the following information: a patient identification code, the implant type and serial number, a short description of the critical condition, the parameter values being in the critical range and date and time of detection. In the preferred embodiment the notification message configuration database comprises information selection data elements identifying the information selected for transmission along with the notification message.

Furthermore, this notification function is configurable to the patient's individual needs and to the physician's general preferences, i.e. the physician may select for each individual patient about which critical situations he wants to be notified. For this purpose the notification message configuration database comprises message trigger identification data elements which identify the situations which shall trigger the transmission of a message.

In it's most preferred embodiment the notification message configuration database is organized such that the physician identification data elements, the message trigger identification data elements and the information selection data elements are assigned to individual patients. The information selection data elements may each or in groups be assigned to a respective message trigger identification data element in order to allow automatic transmission of different information depending on the situation which triggers the transmission of the notification message.

It is to be understood, that the program stored on the data storage means can be further adapted to perform all the functionality described before.

The invention shall now be further disclosed based on an exemplary embodiment together with the figures.

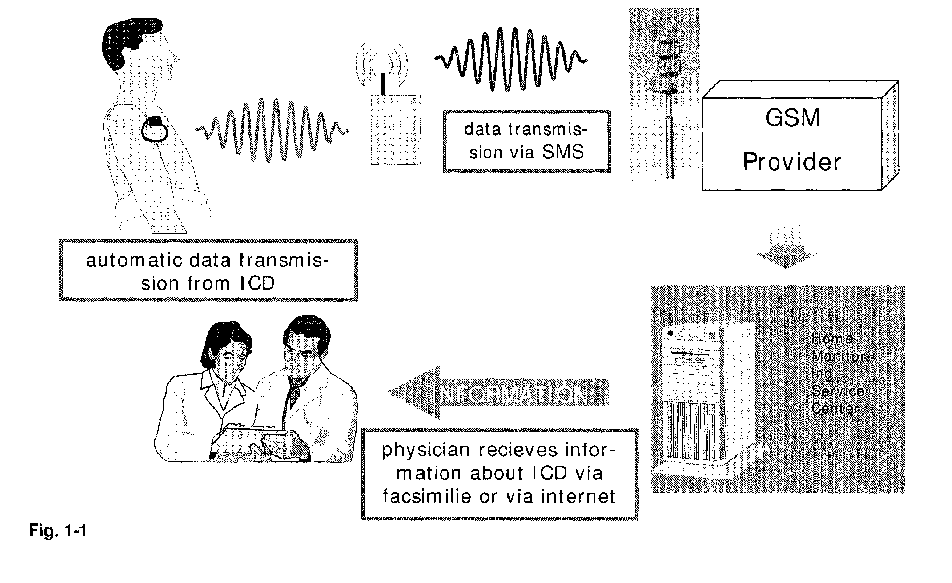

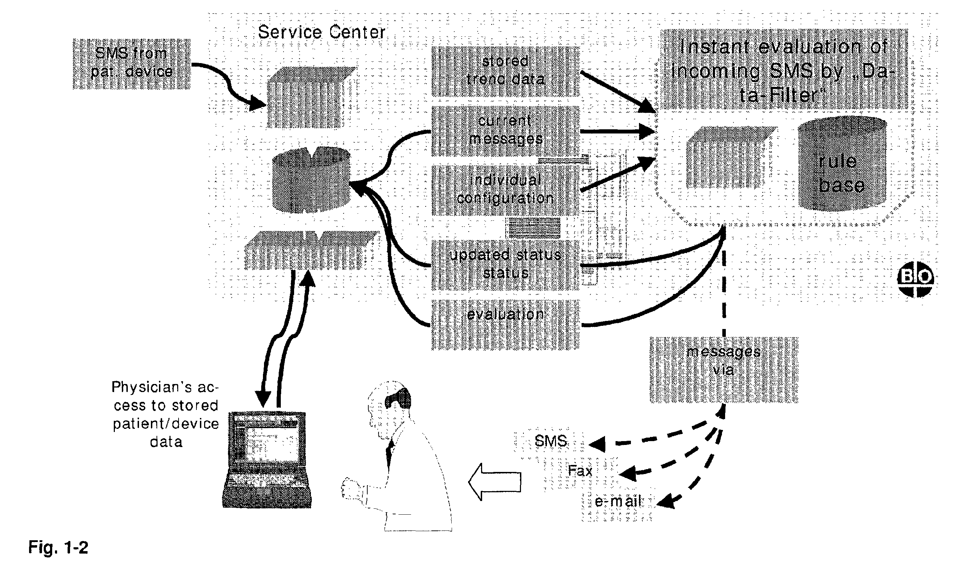

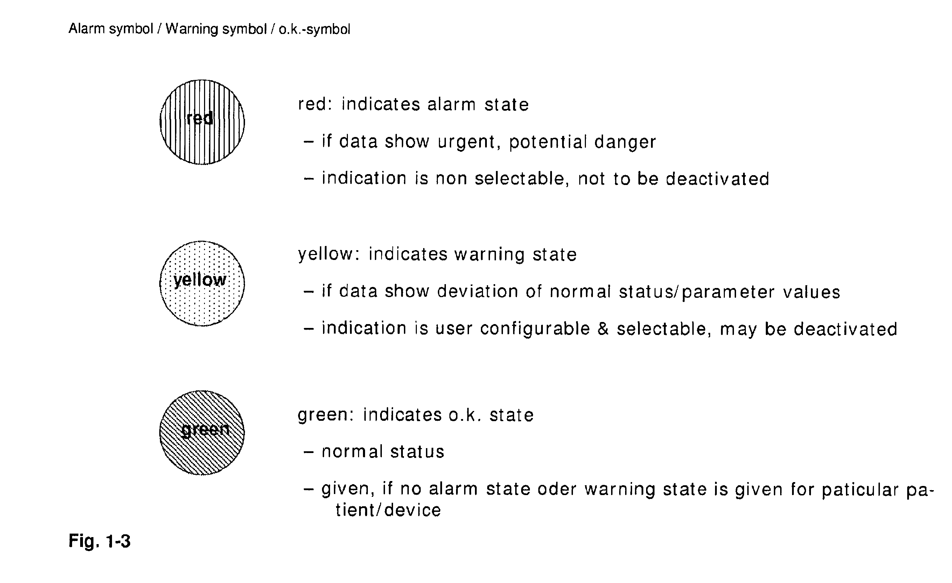

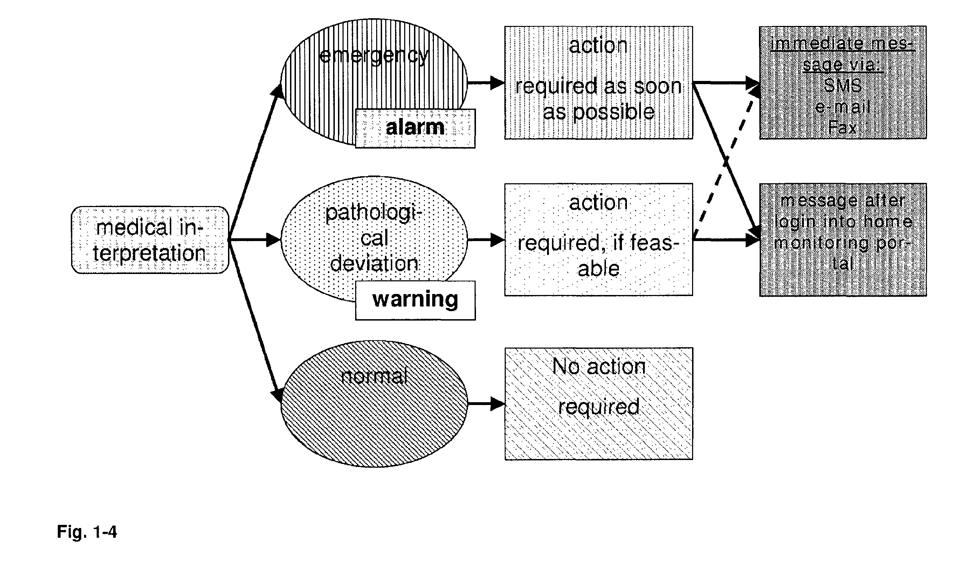

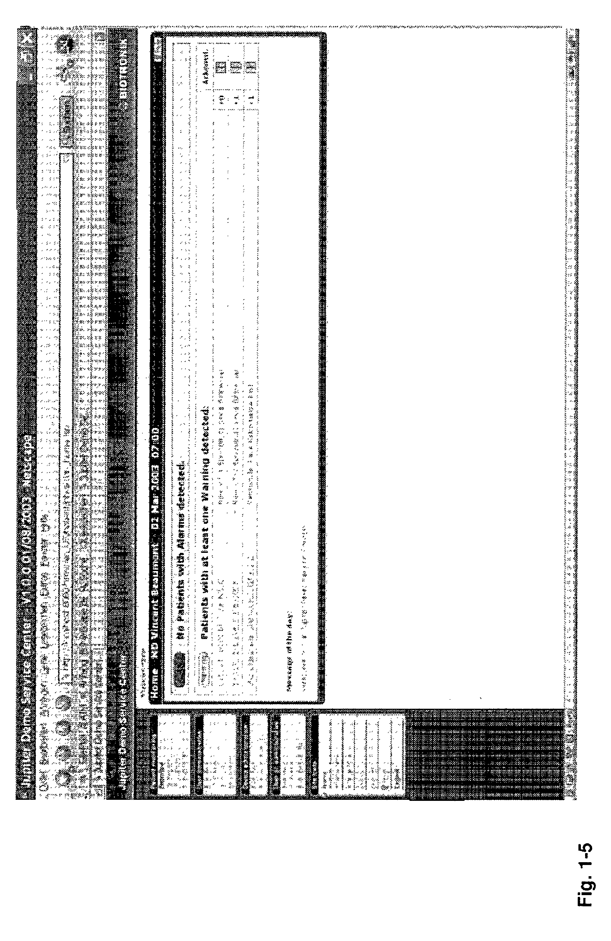

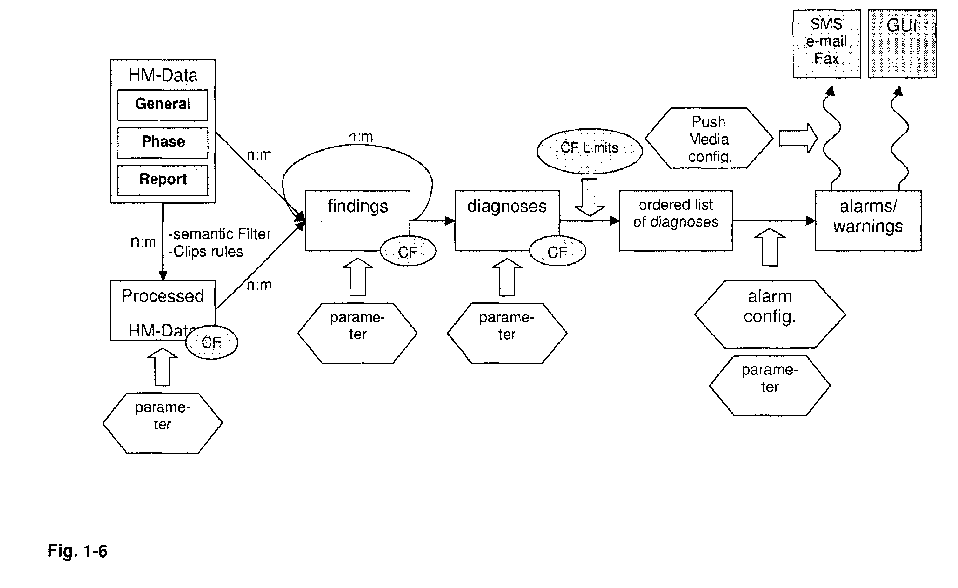

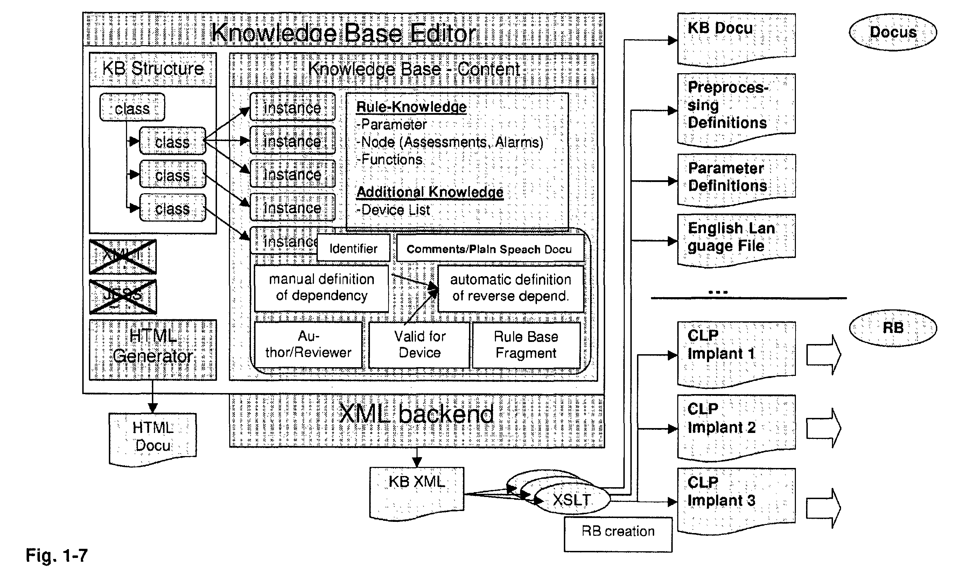

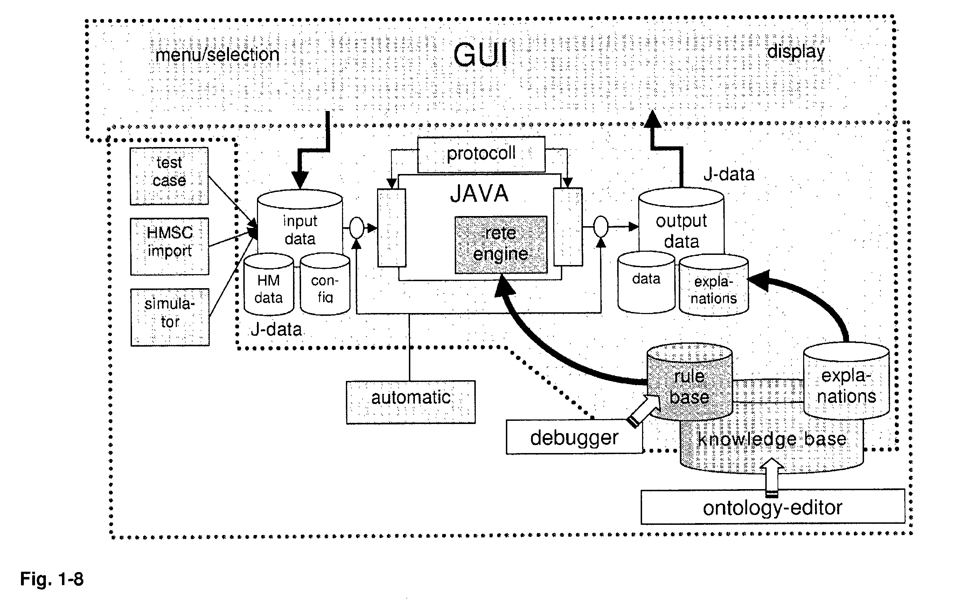

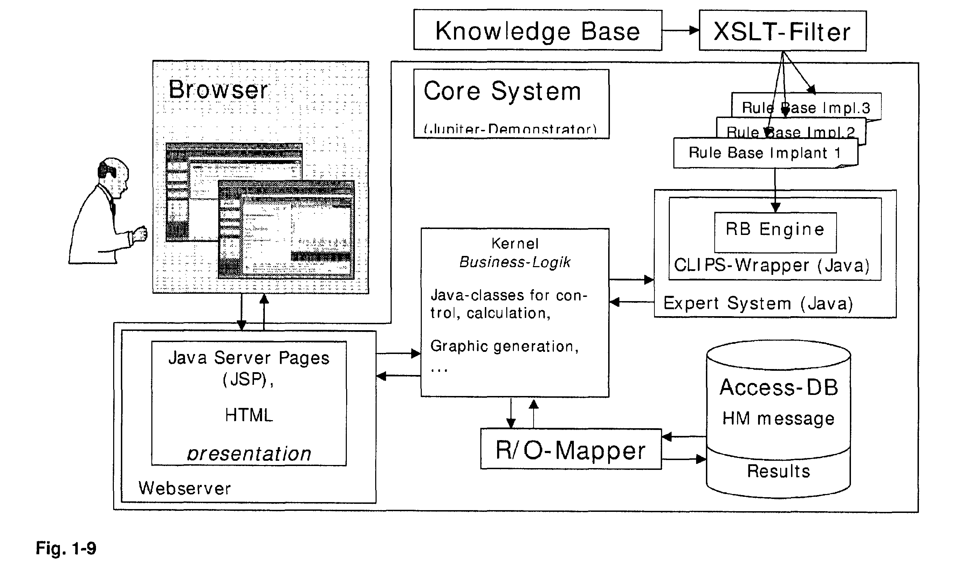

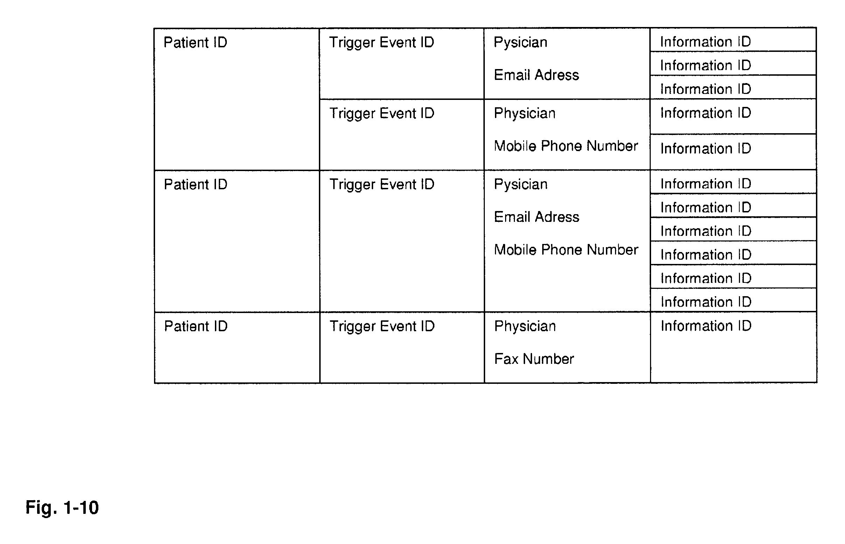

- Figure 1-1:: an overview of a home monitoring system for a patient's health care;
- Figure 1-2:: an overview of the data management system as part of the home monitoring system shown in figure 1;
- Figure 1-3:: the three main status indicator symbols;

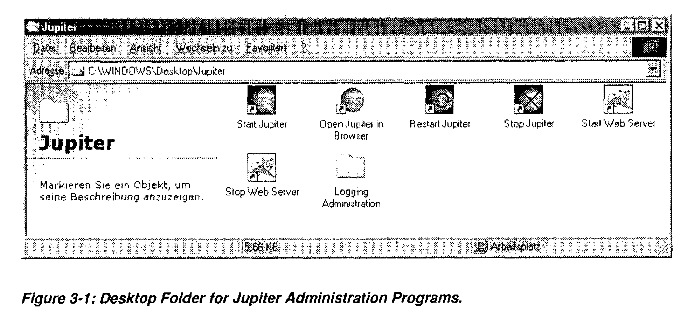

- Figure 1-4:: a rough overview of the rules for generating and the meaning of either one of the symbols shown in figure 3;
- Figure 1-5:: an overview of a graphical user interface provided by the data management system for a physician;
- Figure 1-6:: a structure of knowledge nodes which form a knowledge base, which in turn provides a basis for a rule base;
- Figure 1-7:: a rough representation of the engine transforming the knowledge base into the rule base;
- Figure 1-8:: an overview of the system components of the data management system;
- Figure 1-9:: an overview of the system architecture of the data management system; and
- Figure 1-10:: an example for a notification message configuration database as a part of a communication unit which is a part of the data management system.

Figure 1-1 shows a patient with an implanted medical device, for example an implantable cardioverter/defibrillator (ICD). The ICD is able to initiate an automatic data transmission to an external device in the close proximity of the patient. The external device (patient device) initiates a data transmission via short message strings using a mobile communication infrastructure according to the GSM standard. The short message strings (SMS-messages) are received by a home monitoring service center (HMSC) where the data content of each short message string is evaluated and stored. The data stored in the home monitoring service center is evaluated and information for a physician is generated which is submitted to the physician via facsimile or via internet.

Figure 1-2 shows the core components of the data management system realized in the home monitoring center from a physicians perspective. The data management system comprises a computer system which provides for a data base and for means to evaluate data stored in the data base. These means for evaluation of data comprise an expert system program which is adapted to evaluate stored data based on rules stored in the rule base. Data evaluation leads to messages submitted to a physician.

The physician has access to the data stored in the data base via an interface, for example a notebook being connected to the service centers computer. The notebook serves as well as an input interface allowing the physician to configure some of the rules in the rule base.

Figure 1-3 illustrates symbols shown to the physician for each patient or device he is responsible for. These symbols will appear on the user interface (i.e. the laptop screen) immediately after login by a physician. A red alarm symbol indicates an alarm state as defined by the rules in the rule base. The rule base is configured in a manner to determine an alarm state if data stored in data base indicates a potential health danger which requires urgent action. The physician can not deactivate indication of alarm symbols.

A different type of symbol is given by a warning symbol which indicates a warning state if the data stored in the data base show a deviation of a normal status or deviation of parameter values from normal values. Via the user interface, the physician is able to configure the indication of warning states. He may deactivate the indication of some warning states.

If neither an alarm state nor a warning state is determined by the data management system based on the rule base, an ok-symbol is shown to the physician.

Figure 1-4 shows the concept of indicating three different states, alarm state, warning state and ok-state, in further detail. In particular, from figure 1-4 it is apparent, that the data management system initiates an immediate message to a physician, if an alarm state is detected. The message will be send as short message string, e-mail or facsimile. The message indicating an alarm status is send even if the physician is not logged in into the data management system. A warning state is only indicated by a warning symbol, if the physician logs into the home monitoring portal of the data management system.

Figure 1-5 shows a screen shot of the user interface indicated to a physician after login into the home monitoring portal.

Figure 1-6 is a representation of the structure of the knowledge nodes which are part of the knowledge base and represent an expert's or a physician's knowledge about potential health complications of a patient or technical complications of a device. The knowledge base is defining the rules for evaluating the data stored in the data base and thereby defines the rule base.

Figure 1-7 does show how the knowledge base defines the rules of the rule base. Please note that in the figure the shortcut RB is used for rule base and KB is used for knowledge base.

Figure 1-8 is a further overview of the system components of the data management system. In particular it is shown, how a Java^{TM} engine generates the information displayed on a graphical user interface (GUI) based on the rules defined in the rule base (RB) and derived from the knowledge base (KB).

Figure 1-9 shows a further overview of the system architecture of the data management system.

Further features and further details of operation of the data management system become apparent from the following Jupiter Demonstrator Manual included herewith.

It is to be noted, that the Jupiter Demonstrator described herein is a simulator showing the functionality and the graphical user interface for a system as claimed with the claims. Those features specific for the simulator and to be changed in a real life system are clearly indicated in the following description.

### Jupiter Demonstrator Manual

### Contents

| | |
|---|---|
| 1 General Concept | 11 |
| 1.1 Basic Idea | 11 |
| 1.2 Differences between the Demonstrator and the intended Real Life System | 11 |
| 1.2.1 Omitted Topics | 12 |
| 1.2.2 Additional Simulation Control | 12 |
| 1.2.3 Administrative Tasks | 13 |
| 2 Graphical User Interface | 13 |
| 2.1 Login Pages | 14 |
| 2.1.1 How to sign in and start | 14 |
| 2.1.2 How to control your simulation | 14 |
| 2.1.2.1 Simulation Control | 15 |
| 2.1.2.2 Case Evaluation Form Reminder | 16 |
| 2.1.2.3 Notification Display | 16 |
| 2.2 Basic Layout | 16 |
| 2.2.1 Header | 17 |
| 2.2.2 Menu | 17 |
| 2.2.3 Content Area | 17 |
| 2.2.4 Icons and Symbols | 17 |
| 2.3 Patient Selection Pages | 19 |
| 2.3.1 Physician's Home: Patients with Alarms and Warnings | 19 |
| 2.3.2 Physician's Overview: All Patients | 21 |
| 2.4 Patient Information Pages | 22 |
| 2.4.1 Basic Layout | 22 |
| 2.4.2 Icons and Symbols | 22 |
| 2.4.3 General Status Page | 23 |
| 2.4.4 Medical Data Pages (Belos DR-T) | 24 |
| 2.4.4.1 Basic Layout | 25 |
| 2.4.4.2 Graphs | 26 |
| 2.4.4.3 Device Status | 29 |
| 2.4.4.4 Leads | 29 |
| 2.4.4.5 Antibradycardiac Pacing | 30 |
| 2.4.4.6 Supraventricular Episodes | 30 |
| 2.4.4.7 Ventricular Episodes | 31 |
| 2.4.4.8 How to get Diagnosis Detafts | 32 |
| 2.5 Archive | 33 |
| 2.6 Configuration | 33 |
| 2.6.1 Alarms | 34 |
| 2.6.2 Warnings | 34 |
| 2.6.3 Special Characteristics | 36 |
| 2.6.4 How to get Information about Alarms and Warnings | 36 |
| 2.7 Help | 36 |
| 2.8 Error Messages | 37 |
| 3 Administration | 38 |
| 3.1 Start Jupiter | 38 |
| 3.2 Stop Jupiter | 38 |
| 3.3 Restart Jupiter | 39 |
| 3.4 Start Browser and Open Jupiter | 39 |
| 3.5 Start the Web Server | 39 |
| 3.6 Stop the Web Server | 40 |
| 3.7 Logging Administration | 40 |
| 4 Technical Background of the Demonstrator | 41 |
| 5 Trouble Shooting | 43 |
| 5.1 What to do if no Help/Details/Info Pages show up? | 43 |
| 5.2 What to do if 'internal program errors' occur? | 43 |
| 5.3 What to do if there is no contact? | 44 |
| 5.4 What to do if nothing works to solve the problem? | 44 |
| 6 Appendix | 44 |
| 6.1 Icons, Buttons, and Symbols | 44 |
| 6.1.1 Status Indicators | 44 |
| 6.1.1.1 General Status | 44 |
| 6.1.1.2 Group Status | 45 |
| 6.1.1.3 Acknowledgement | 45 |
| 6.1.2 Configuration Selection | 46 |
| 6.1.2.1 Warnings | 46 |
| 6.1.2.2 Groups | 46 |
| 6.1.3 Symbols in Graphs | 46 |
| 6.1.4 Buttons | 47 |
| 6.1.4.1 Anywhere | 47 |
| 6.1.4.2 Simulation Control | 47 |
| 6.1.4.3 Status Display | 47 |
| 6.1.4.4 Time Resolution for Graphs | 48 |
| 6.1.4.5 Configuration | 48 |
| 6.2 Diagnostic Messages | 48 |
| 6.2.1 Alarms | 48 |
| 6.2.1.1 Group Device Status | 48 |
| 6.2.1.2 Group Lead Failure | 52 |
| 6.2.1.3 Group Ventricular Therapies | 56 |
| 6.2.2 Warnings | 56 |
| 6.2.2.1 Group Device Status | 56 |
| 6.2.2.2 Group Leads | 60 |
| 6.2.2.3 Group Antibradycardiac Pacing | 64 |
| 6.2.2.4 Group Supraventricular Episodes | 67 |
| 6.2.2.5 Group Ventricular Episodes / Detection | 68 |
| 6.2.2.6 Group Ventricular Episodes / Therapy | 75 |
| 6.2.3 Special Characteristics | 81 |
| 6.2.3.1 Group Leads | 81 |

### 1 General Concept

### 1.1 Basic Idea

Compared to previous times, Home Monitoring gives the physician a lot of additional information about the state of a patient. However the examination of the Home Monitoring data is very time consuming. Therefore it is necessary to assist the physician by doing automatic evaluation. This relieves the physician of routine jobs and helps him to concentrate on critical cases.

The Jupiter project aims at developing a tool for automatic evaluation of Home Monitoring data based on the concept of the existing Home Monitoring Service Center II (HMSC II). According to a well-known pattern, the tool will classify the evaluation results by three categories, the so-called Monitoring Status:
- No finding,
- Warning,
- Alarm.

To allow access to the evaluation results as easy as possible, the tool is based on internet technology, i.e. the physician views and examines the data by means of a web browser.

The presented Jupiter Demonstrator uses the global appearance of a Home Monitoring Service Center (HMSC) user interface but it introduces new design elements and additional information due to its evaluation of the Home Monitoring data. Nevertheless, it is not intended to expand the HMSC directly. The Jupiter Demonstrator should rather be viewed as a prototype for proof of concept and feasibility test.

### 1.2 Differences between the Demonstrator and the intended Real Life System

The Demonstrator serves to show just the basic points of evaluation and thus works only as a kind of simulation program. Therefore it differs from a fully-fledged Home Monitoring system in some ways.
→ Convention: Wherever this finger symbol is used, the manual points out a specific difference between the Demonstrator and the intended real life system.
→ There are some topics that are omitted due to Jupiter's concentration on evaluation. Other topics that are not needed in a real life system are added to control the simulation and the Jupiter Evaluation Study. Yet another difference arises from the different environment the Demonstrator and the real life system are meant to run in.

### 1.2.1 Omitted Topics

This prototype software focuses on the interpretation, evaluation and presentation of the transmitted Home Monitoring data. Therefore some topics that you may find in the current HMSC II interface are disabled:
- User administration: Even if the administration menu is displayed most of the menu items are disabled.
- Live data input: The simulation works with stored Home Monitoring data. These data are derived from real messages which are fine-tuned to increase the density of detected alarms and warnings for the simulation and to preserve anonymity.
- Notification: No notification by e-mail, SMS, or fax in case of alarms or warnings is done. Rather a message is displayed on a page that is specific for the simulation.

### 1.2.2 Additional Simulation Control

To provide the user with the means to compare different outcomes of the automatic evaluation for different settings and to support studies to evaluate the quality of Jupiter the following topics are added:
- Simulation control center: On a real life system, the Sign-in Page will lead directly to a web page. In the Demonstrator there is an intermediary page. On this page the would-be notifications are displayed and the simulation date can be incremented or reset.
- Just-in-time evaluation: The evaluation results are not pre-calculated. On a real life system this will be done when a Home Monitoring message arrives. On the Demonstrator this is done when the simulation date is incremented. This is necessary because the user can change the configurations which influence the outcome of the calculation. Regrettably, the just-in-time evaluation introduces a delay that will not persist once Jupiter is integrated in a real life system.
- Deactivation of patients: To define an end for projected studies the evaluation of patients is individually ended after a defined period of time.

To better understand how the simulation can be controlled, please consult 2.1.2.

### 1.2.3 Administrative Tasks

Because the Demonstrator is intended to run stand-alone, a local web server is necessary. On a real life system this server runs at BIOTRONIK and the user will need nothing more than a web browser. The same holds true for the database. If you are interested in more information on the technical background, please consult chapter 4.

In general, the additional administration tasks involve only starting and stopping of the installed web server. For more information on this, see chapter 3.

### 2 Graphical User Interface

The physician accesses the patients' data through a common web browser. These data consist of the transmitted Home Monitoring data and Jupiter's evaluation results. 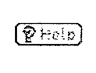 The following chapters describe all the web pages of the Jupiter Demonstrator and their intended use. Most of the descriptions are also available by clicking on the 'Help' button of each web page. 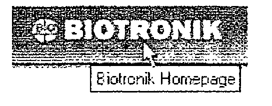 If you require a quick and short help on the meaning of a button, a link, or a symbol, just try to view the so-called tool tip by setting the mouse cursor on it.
→ For the correct presentation of Jupiter's web pages, it is strongly recommended to use Netscape Version 7.1 as your browser. Of course, the intended real life system will support all common web browsers.

### 2.1 Login Pages

The Demonstrator includes sort of two consecutive start pages:
- the Sign-in Page (the real one) and
- the Simulation Control Page, that is a second one to set simulation values before entering the demonstration system.
   → On a real life system, only the first Sign-in Page is used.

### 2.1.1 How to sign in and start

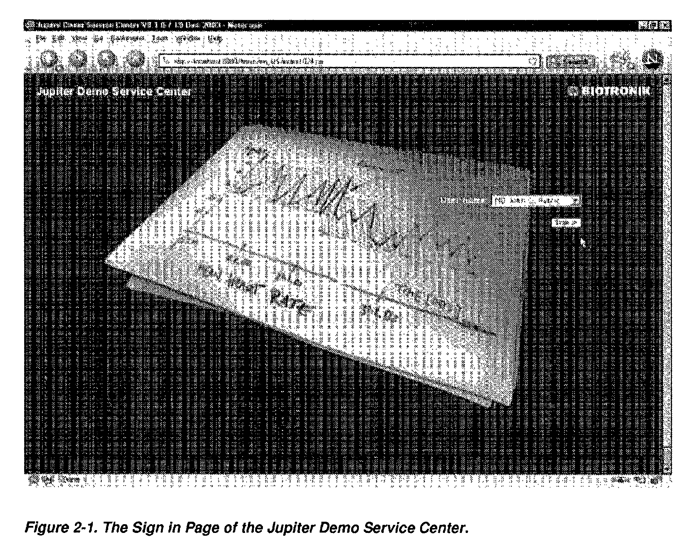

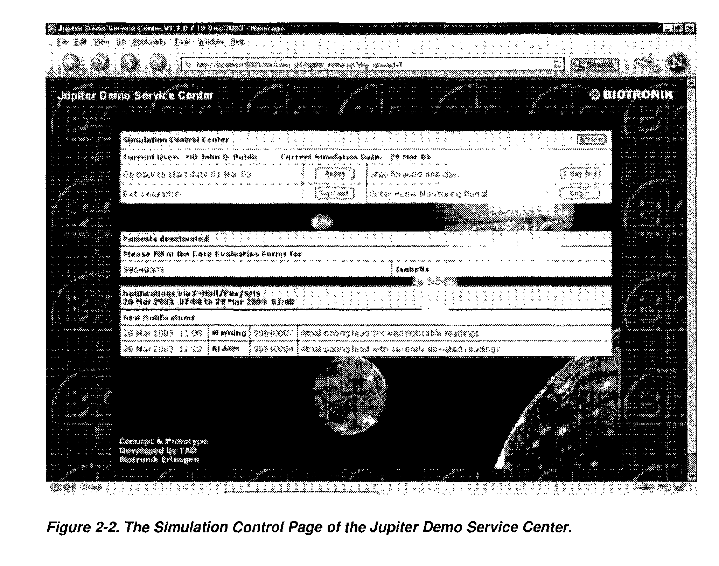

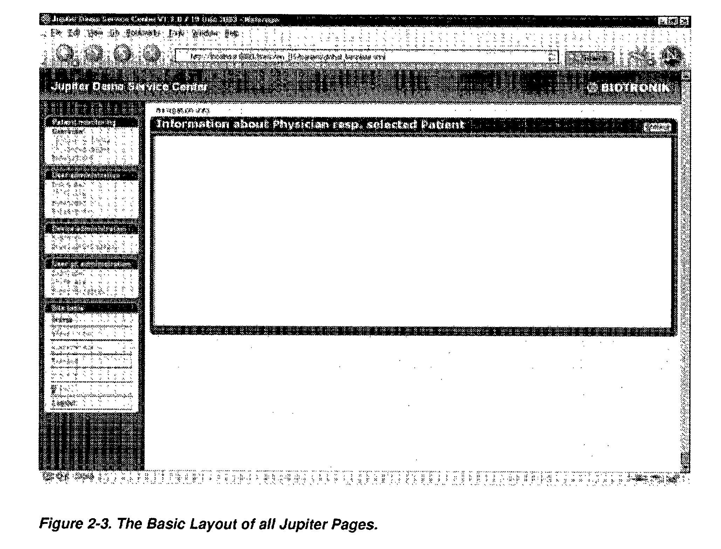

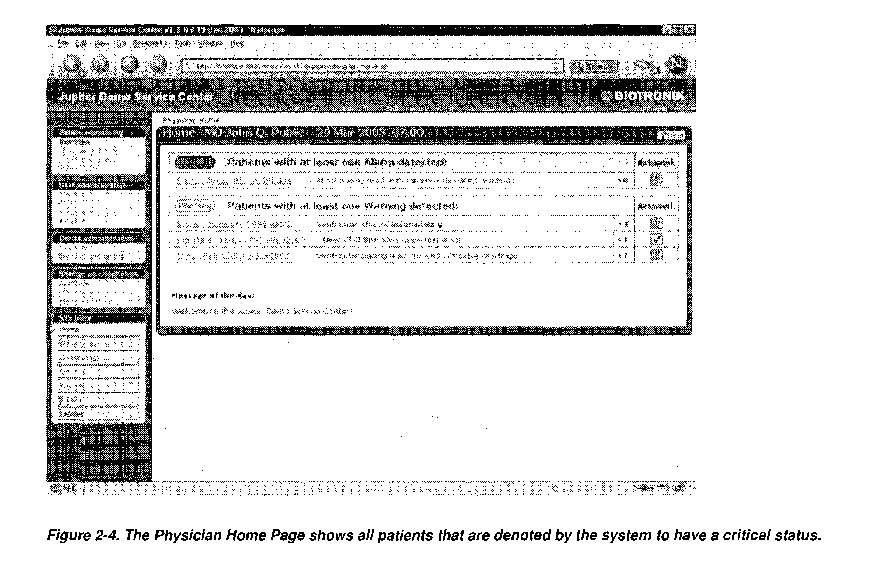

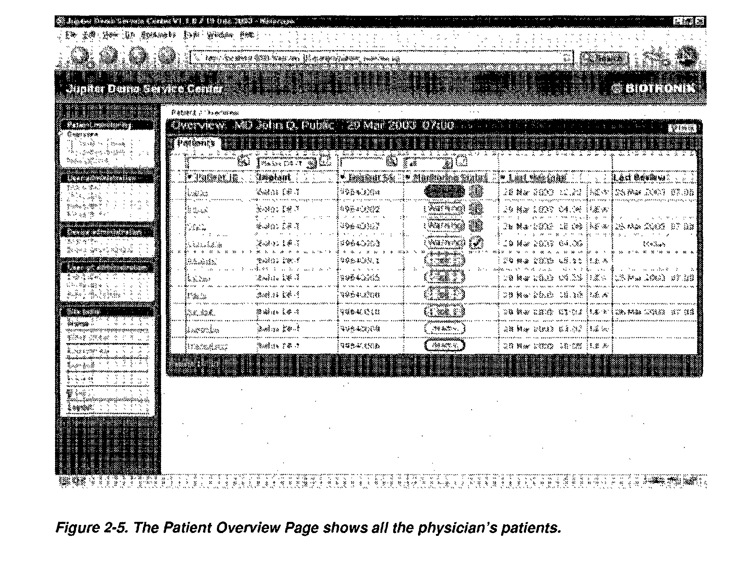

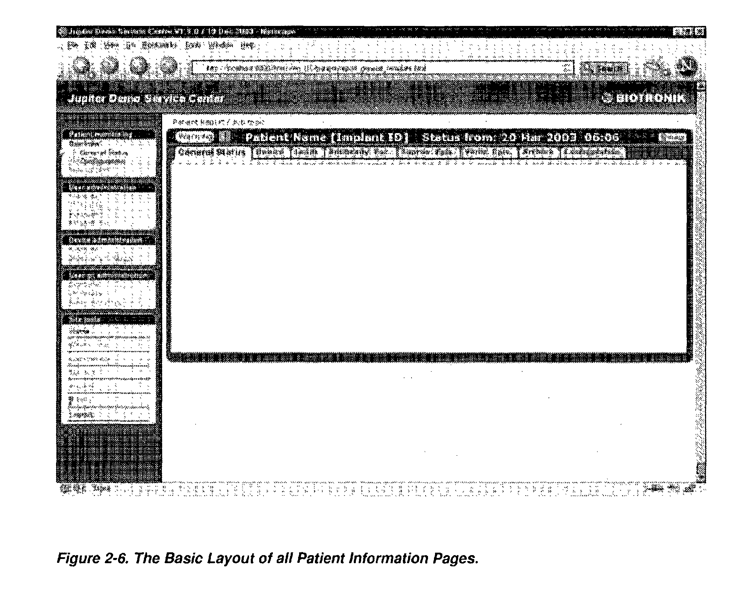

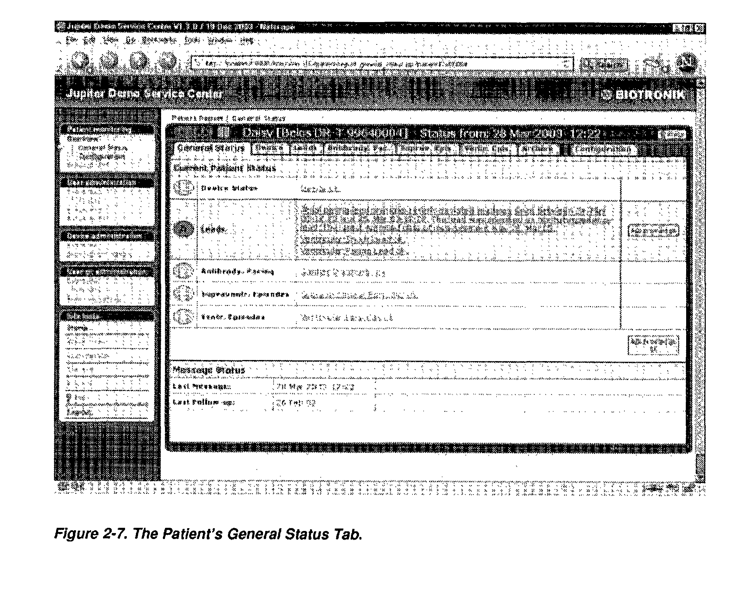

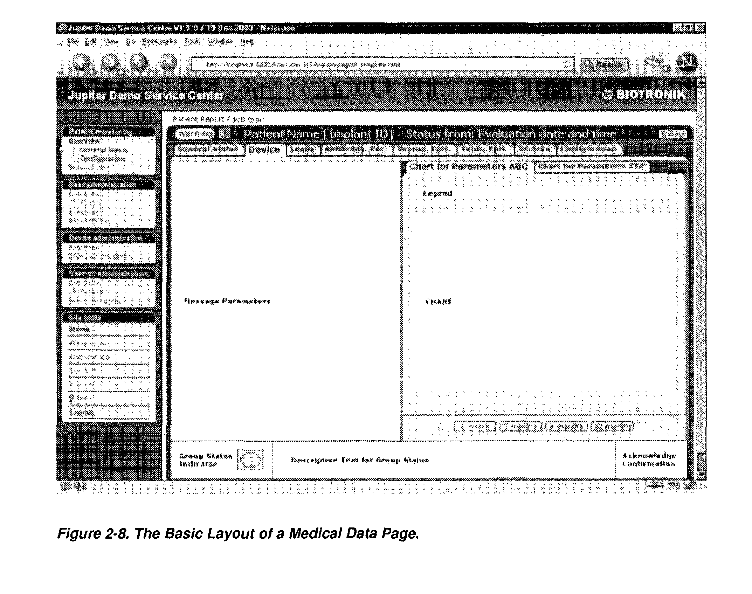

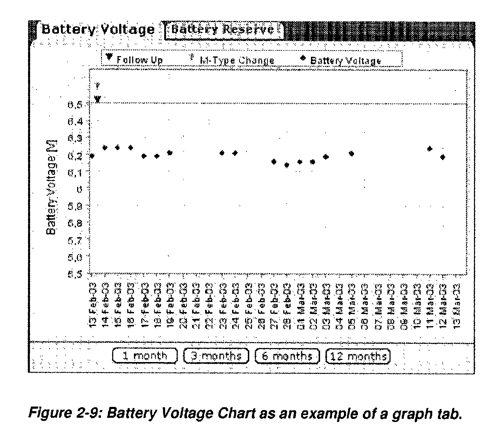

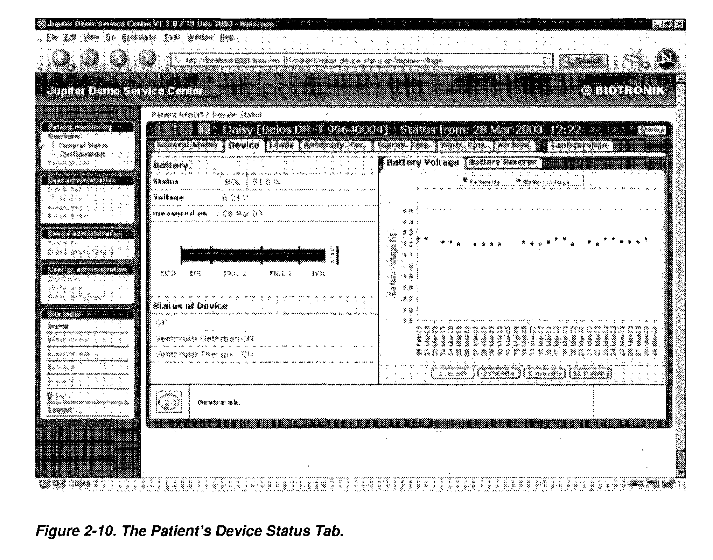

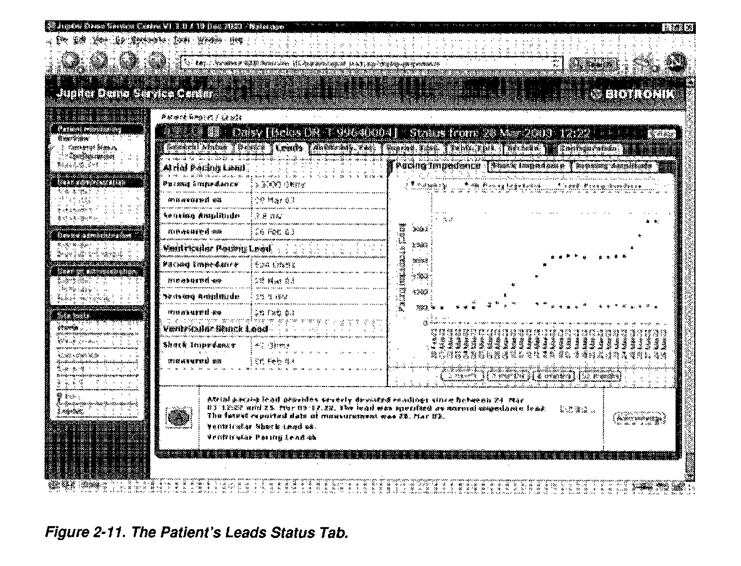

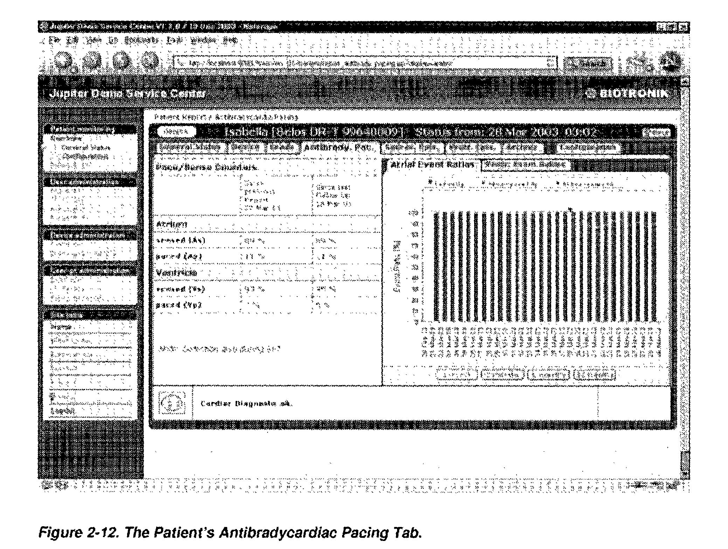

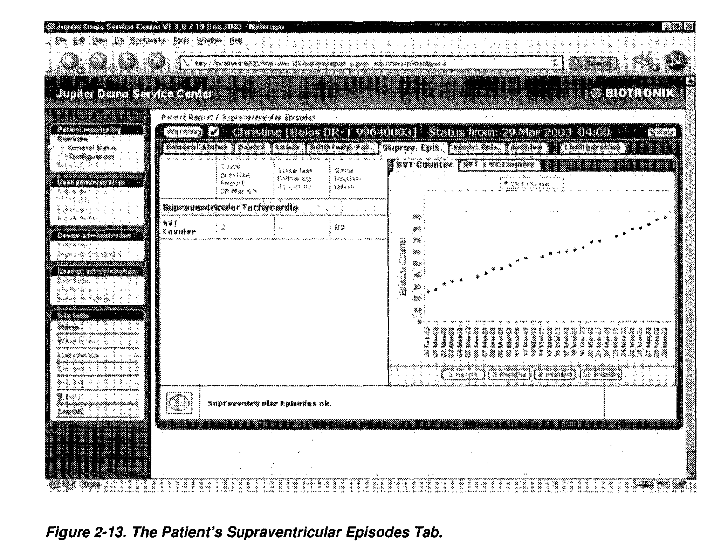

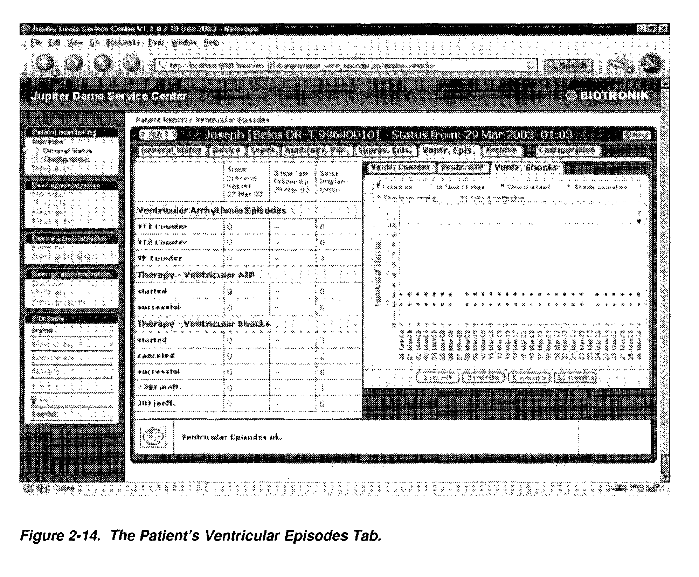

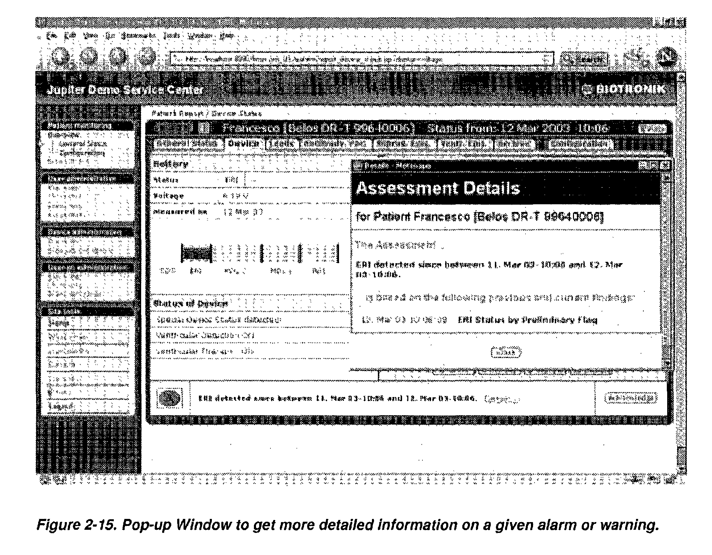

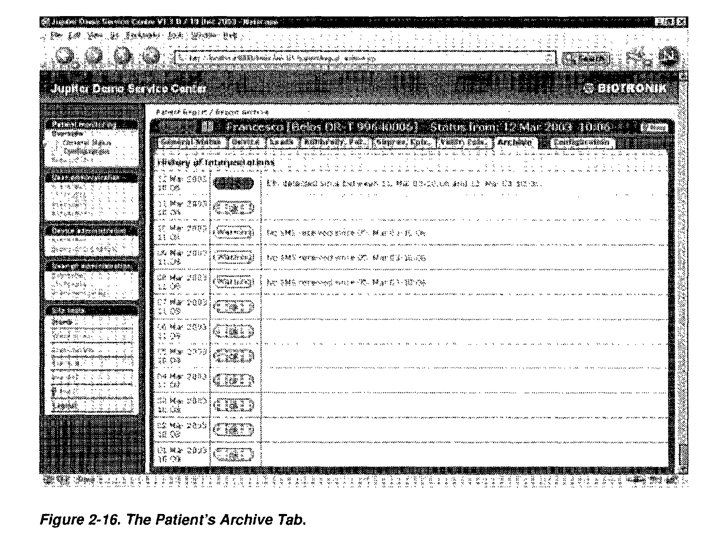

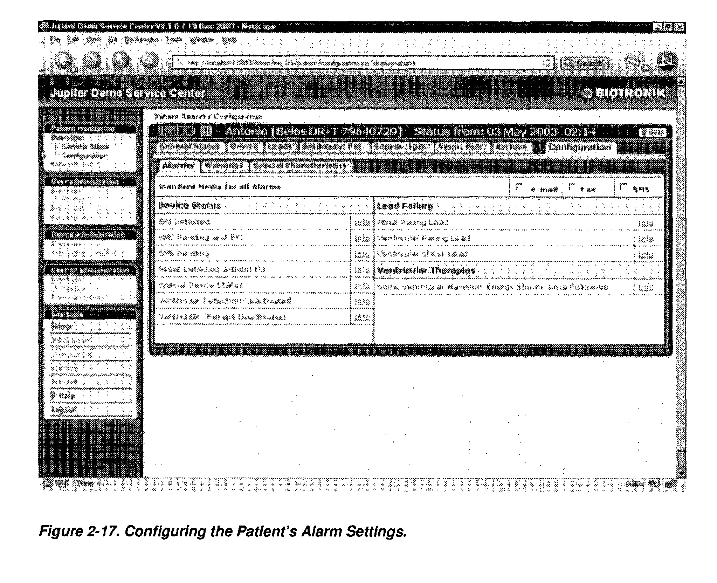

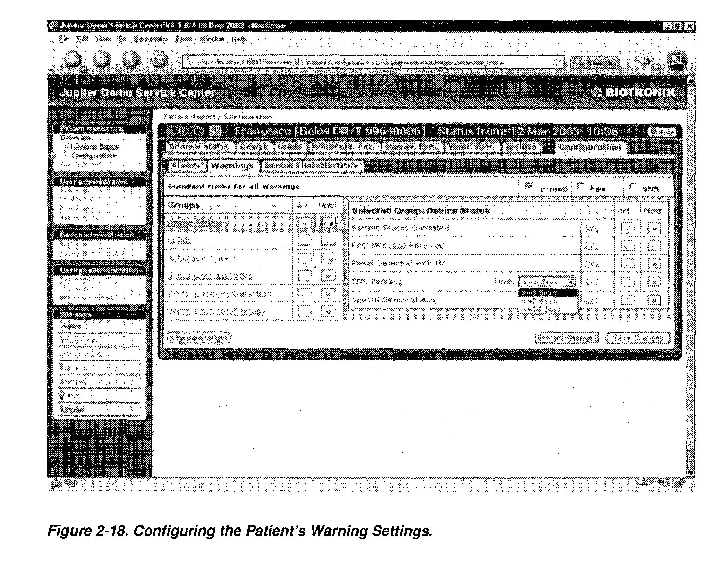

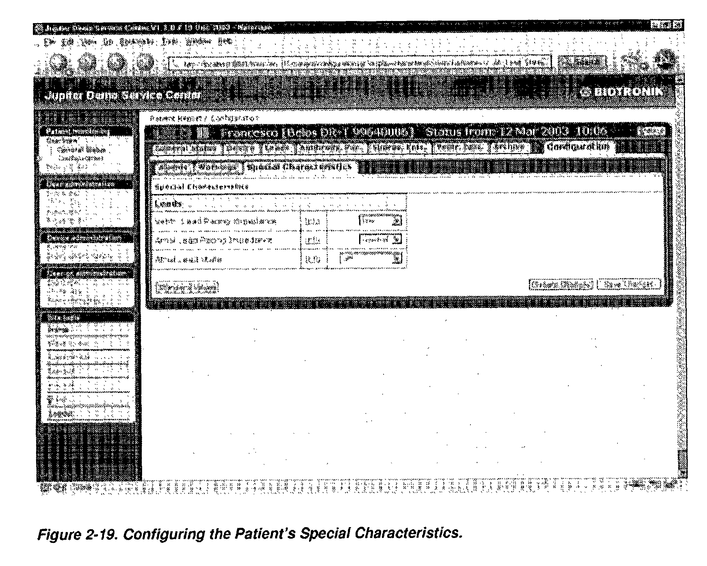

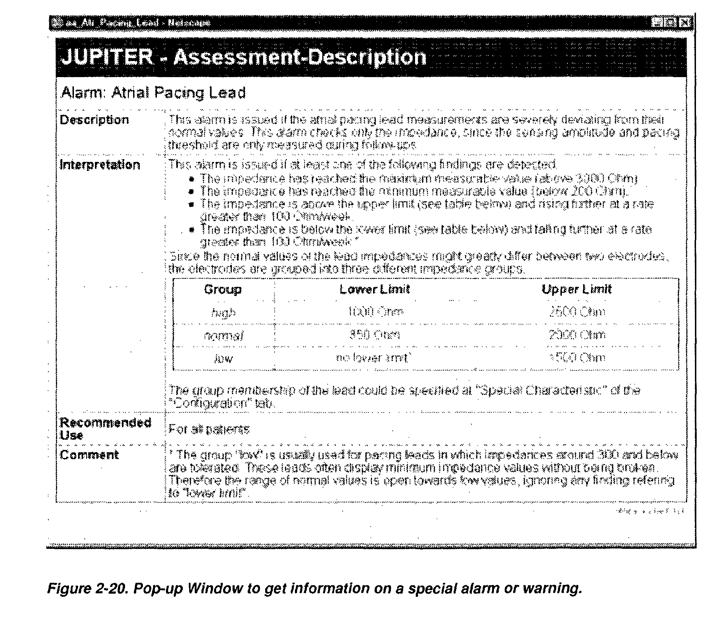

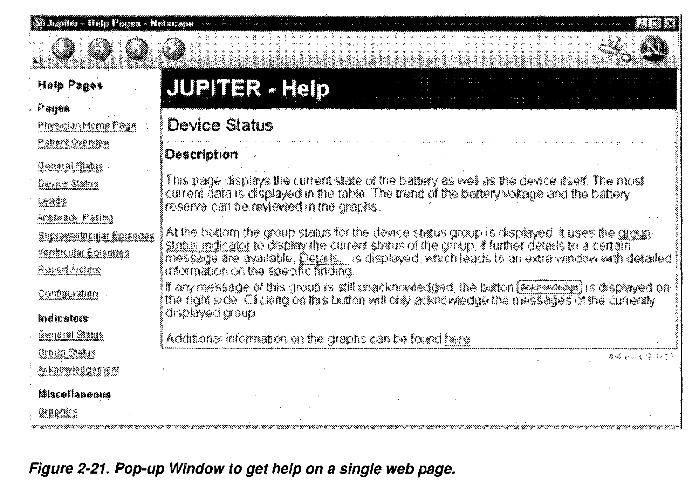

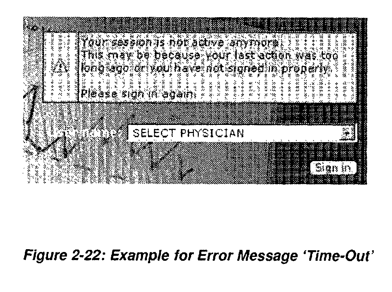

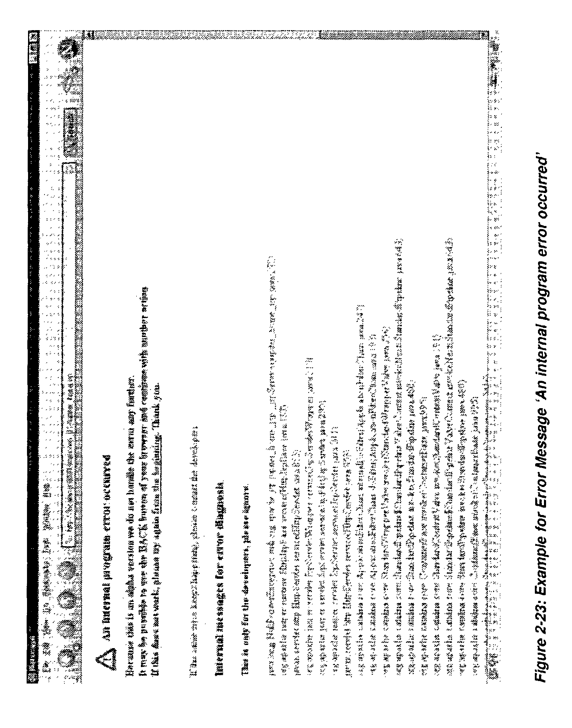

See Figure 2-1 shows the Sign-in Page where you just have to select the physician ID that is to be used for the simulation resp. for the Jupiter Evaluation Study (JES). A list of predefined physicians is already given. Please press the 'Sign in' button to enter the Jupiter Simulation System.
→ On a real life system, the login would require information like user group, user name, password, or something similar.

### See Figure 2-1. The Sign in Page of the Jupiter Demo Service Center.

### 2.1.2 How to control your simulation

After sign-in a second 'Login' page is build up (see See Figure 2-2). It is called the Simulation Control Page because it combines all simulation functions, which are not part of the later internet based system. It is used to control the simulation and displays messages that either are specific for the Jupiter Evaluation Study or replace functions like notifications via fax, SMS, or e-mail.

The screen is divided into three parts:
- the Simulation Control Center
- the Case Evaluation Form Reminder (displayed only if necessary)
- the Notification Display
   → Attention: This page is only necessary for the Demonstrator. The real life system will allow the user to directly enter the physician home page after identification.

### See Figure 2-2. The Simulation Control Page of the Jupiter Demo Service Center.

### 2.1.2.1 Simulation Control

The four buttons of the Simulation Control Center have the following functions: 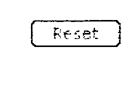 This resets the complete simulation to the start date, which is specific for every physician and displayed at the left of the button. The reset will discard all actions performed, including the evaluation done so far and the acknowledgements. However, the settings of the individual configurations of the patients are preserved. Before the reset is really done the user will be asked one more time. 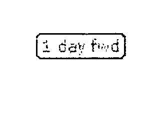 The simulation is advanced by 24 hours. The Jupiter system will evaluate all messages, which would have been received during this time. Depending on the system, the evaluation will take some time. The real life system will perform these calculations in the service center as soon as the message is received and the user will not experience this delay. 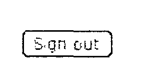 This will return to the Sign-In Page, which allows to select a physician. The state of the simulation will be preserved until the next sign-in. 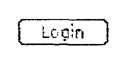 This will enter the Jupiter pages. The Physician Home Page with the essential cases will be displayed next.

### 2.1.2.2 Case Evaluation Form Reminder

This part tells you for which patient the simulation has ended. If a patient is displayed here, it would be an excellent time to fill out the Case Evaluation Form for this patient. For safety reasons the patient is displayed on 3 consecutive simulation days. This table is only visible if there is actually a patient to be displayed.

A patient, for whom the simulation has ended, will be marked as 'deactivated' in the following Jupiter pages.

### 2.1.2.3 Notification Display

This table substitutes the notifications, which would normally be sent out by fax, e-mail, or SMS according to the user's preferences. It displays all notifications, which would have been sent in the time period specified (usually the last 24 simulation hours). It states the date and time of the notification, the serial number of the patient's device, the priority level, and a short description of the found assessments.

### 2.2 Basic Layout

All of the following Jupiter pages base on the layout depicted in See Figure 2-3.

### 2.2.1 Header

The header shows the logos of the Jupiter project and of BIOTRONIK including a link to the BIOTRONIK Homepage (available only if internet access is supported).

### 2.2.2 Menu

The menu on the left serves to navigate to the most important pages.
→ The pale-grey items denote functionalities that deal mainly with administration tasks. As already pointed out (see 1.2.1), those items are disabled, i.e. the Jupiter Demonstrator does not support administrational functions.

The 'Logout' item leads back to the Simulation Control Page.

### 2.2.3 Content Area

The content area consists of an own header where the name of the selected physician resp. the identifier for the patient is displayed. At the right edge you find the button for the context help.

The remaining area displays information depending on the selected page.

### See Figure 2-3. The Basic Layout of all Jupiter Pages.

### 2.2.4 Icons and Symbols

A metaphor that is used all over Jupiter is the color scheme of traffic lights. The icons that may point out the patient's general state on any Jupiter page are as follows: 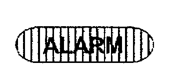 Red means that a serious problem, an alarm, is detected. 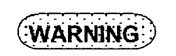 Yellow means that a less serious problem, a warning, is detected. 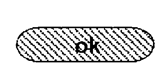 Green means that no abnormality (neither an alarm nor an activated warning) is detected.

Please note that for reproduction purposes in this text the heraldic color scheme used in the representations. That means that the hatching represents plain colors an a users screen. A vertical hatching stands for the color red, a dotted hatching represents yellow and an oblique hatching means green. Characters within the symbols should support color-blind users and proper print-out.

The color scheme is also used for deactivated patients. Here the frame color denotes the last status before deactivation:

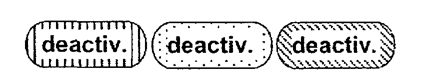

In rare cases no status is available. In general, this means that evaluation is not yet done because the simulation has not yet started. This status is denoted by a grey icon:

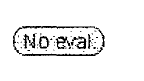

If the service center has never received any messages from a patient's implant just a short textual note 'No Messages' is displayed.

The physician must actively acknowledge that he took notice of an alarm or warning. Two additional icons indicate the acknowledge status of a patient:

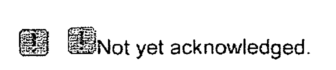

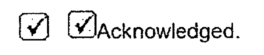

The following chapters give a more detailed introduction of each web page. You will get more information on the used symbols and buttons or how to acknowledge alarms or warnings.

### 2.3 Patient Selection Pages

There are two pages to select a special patient:
- the so called Physician Home Page and
- the Patient Overview Page.

The header of both content areas displays
- the name of the page, e.g. 'Physician Home or 'Patient / Overview'.
- the full name of the selected physician, e.g. 'MD John Q. Public',
- the simulated login date and time, e.g. '29 Mar 2003 07:00', and
- the 'Help' button at the right edge.

### 2.3.1 Physician's Home: Patients with Alarms and Warnings

### The Physician Home Page (see

***See Figure 2-4***) is the very first page that is displayed after the physician's login. It displays all patients for which at least one alarm or at least one activated warning has been detected.

This page can always be reached by clicking the item 'Home' in the service center menu bar on the left side of any data page.

The page brings up two tables: 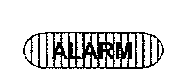 The upper table displays all patients with at least one detected alarm. For these patients all alarm messages are displayed. 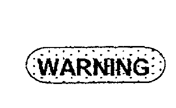 The lower table displays only patients with detected activated warnings but without any alarm. For these patients only the warning with the highest priority is displayed. 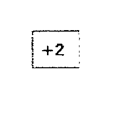 In both tables the number of detected activated warnings that are not displayed on this page, is specified.

### See Figure 2-4. The Physician Home Page shows all patients that are denoted by the system to have a critical status.

The following rules apply for this page:
- Detected Alarms are always displayed. Patients without any alarm or activated warning or patients without evaluation do not show up on this page.
- A patient is no longer evaluated when his simulation period has ended.
- Deactivated warnings are ignored on this page even if they are detected. Please consult 2.6.2 for information on how to configure warnings to be disabled.
- It is possible that more warnings are displayed here than are marked to have been pushed on the Simulation Control page. This is because notification for a warning can be activated independently of the activation of this warning.

On the right hand side of each table the acknowledgement status of the patient is displayed. The following two symbols are used as indicators: 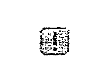 Not yet acknowledged, i.e. at least one alarm or activated warning has not yet been acknowledged. Reviewing the data and acknowledging the messages is recommended. 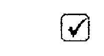 Acknowledged, i.e. all detected alarms and activated warnings have been acknowledged.

The acknowledgement of detected alarms and activated warnings is the "feedback" from the physician to the Home Monitoring Center. It signals that the alarm or warning message has been read.

However, please notice that it is not possible to acknowledge an alarm or warning on this page. Rather the physician has to select the patient first and read further details on the alarm or warning before acknowledgment. See 2.4.3 for information on how to acknowledge alarms or warnings.

### 2.3.2 Physician's Overview: All Patients

The table on this page comprises all patients assigned to the currently logged-in physician (see See Figure 2-5). This includes active patients as well as inactive patients.

This page can always be reached by clicking the item 'Overview' in the service center menu bar on the left side of any data page.

### See Figure 2-5. The Patient Overview Page shows all the physician's patients.

The columns 'Patient ID', 'Implant' and 'Implant SN' just display what they stand for.

The column 'Monitoring Status' displays the current status of each patient using the general status indicators as well as the acknowledge indicators that have already been introduced.

The column 'Last Message' displays the date of the latest received message prior to the current (simulation) date. If a message is received after the last review date, the indicator 'NEW' is displayed. The review date is automatically updated as soon as the general status page of a patient (see 2.4.3) has been displayed during the session.

To view the individual data of a patient (see 2.4), the user can click on the patient ID or the general status indicator.

The controls at the top of some columns are used to filter the displayed patients. However, this feature is not supported by the Jupiter Demonstrator. The sorting of the table by clicking on the column header is activated for: patient ID, implant serial number, monitoring status and last message.

### 2.4 Patient Information Pages

These pages show information about the patient's current status.

Roughly spoken, all of the assessments that contribute to the general status can be classified as discrete groups like status of device, status of leads, detection of atrial/ventricular episodes and other domains.

### See Figure 2-6. The Basic Layout of all Patient Information Pages.

### 2.4.1 Basic Layout

All patient information pages base on the layout shown in See Figure 2-6.

The header of the content area displays
- the icons that indicate the general status and their acknowledgement status,
- the patient's identifier and the device identification,
- the date of evaluation, that is in general the date of the last device message,
- the 'Help' button at the right edge.

The tabs below the header lead you to the denoted groups. In general, the name and the number of those groups and tabs depend on the patient's implant type. The selected tab is always highlighted.

The remaining area displays information depending on the selected tab.

### 2.4.2 Icons and Symbols

As already mentioned, the complete status of the patient, also known as the general status, is divided into different sub groups. Each group has its own tab. Furthermore each group displays a group status indicator as well as a textual description of its current state. So for every group an own group status is displayed by one of the following symbols: 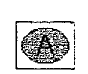 At least one alarm has been detected in this group. 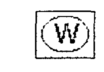 At least one activated warning has been detected in this group. This group has no alarms.

Unlike with the general status, a group can have two sorts of OK. 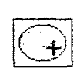 At least one deactivated important warning has been detected in this group. This group has no alarms or activated warnings. 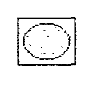 This group has no alarms nor activated warnings nor deactivated important warnings detected.

If there are no evaluations available, e.g. at the first day of simulation, the following grey symbol is shown: 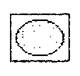 No evaluations are available, e.g. at the first day of simulation.

### 2.4.3 General Status Page

This page displays the general status of the patient individually for each of these 5 groups (see See Figure 2-7):
- Device Status
- Leads
- Antibradycardiac Pacing
- Supraventricular Episodes
- Ventricular Episodes
   → Remember that the name and the number of the assessment groups would be specific for a device type. The Demonstrator deals only with the Belos DR-T implant.

### See Figure 2-7. The Patient's General Status Tab.

The General Status Page is the one that is reached when a patient is selected either from the physician's home or from the overview page. When examining a patient it can also be reached by the enabled 'General Status' item from the left menu bar.

At least this page must be viewed to update the review date for this patient that is shown on the patient overview page (See Figure 2-5).

Each group displays a group status indicator as well as a textual description of its current state. The used symbols have already been introduced (see 2.4.2).

The individual pages of each group can be reached either by clicking on the specific message or by using the tabs at the top of the screen.

The detected alarms and activated warnings can be acknowledged either group wise by clicking the button 'Acknowledge' to the right or for all groups simultaneously by clicking the button 'Acknowledge All'.

Please note that deactivated warnings or normal findings ("ok") require no acknowledgement.

### 2.4.4 Medical Data Pages (Belos DR-T)

The medical data pages display the (device-specific) medical data that were measured by the implant, transmitted via the patient device to the Service Center and evaluated by the Jupiter system for each group of assessments. Regarding Belos DR-T patients, focus is put on the five different groups already mentioned:
- Device Status
- Leads
- Antibradycardiac Pacing
- Supraventricular Episodes
- Ventricular Episodes

Each of the groups is represented by a single tab.

### 2.4.4.1 Basic Layout

All medical data pages base on the layout that is shown in See Figure 2-8.

The left side of the content area lists the values of the specific message parameters that are valid for the last message.

On the right side you can select a chart tab that allows you to investigate the previous trend of one or more parameters. The highlighted tab at the top of the plot denotes which parameters are currently displayed. The dates are written at the bottom of the graphs. The last message is always displayed on the right side of the plot.

### See Figure 2-8. The Basic Layout of a Medical Data Page.

At the bottom of the page the group status for the selected group is displayed. It incorporates the group status indicator and a short textual description of the findings. If further details to a certain assessment are available, a link to 'Details...' is displayed, which leads you to a pop-up window with detailed information on the specific finding (see 2.4.4.8).

If any message of the selected group has not yet been acknowledged, the button 'Acknowledge' is displayed on the right side. Pressing this button will acknowledge only the messages of the currently displayed group.

### 2.4.4.2 Graphs

In addition to the ICD parameters, special information on the device and its counters is displayed. This is necessary because follow-ups, service updates and different counter resets may influence the parameter values received from the ICD. Those special 'events' are marked in the white header of the plots by colored array-down symbols.

All the markers and symbols that are used in the graphs signal the type, date, and time of the message. A short name for the used symbols is given in the legend of the graphs.

### See Figure 2-9: Battery Voltage Chart as an example of a graph tab.

### Special Symbols

| **S** | **Name** | **Description** |
|---|---|---|
| ▼ | Follow Up | A follow-up was detected in the corresponding message. |
| ▼ | Device Reset | A complete device reset was detected in the corresponding |
| | | message. |
| ▼ | M-Type Change | The content of the messages (SMS) may change depending |
| | | on the firmware version of the device. This symbol signals that |
| | | a change in the message content was detected in the |
| | | corresponding message. It affects the internal interpretation of |
| | | certain flags. |
| ▼ | Event Counter Reset | The symbol signals a reset in event counters of sense and |
| | | pace events. This may occur after reprogramming or together |
| | | with a complete device reset. |
| ▼ | Tachy Counter Reset | This symbol signals a reset of the tachy counters, e.g. |
| | | together with a complete device reset. All episode and therapy |
| | | counters are set to 0. |

### Group-sorted ICD Parameters (Belos DR-T)

| **S** | **Name** | **Unit** | **Group/Chart Tabs** | **Date Axis** **(abscissa)** |
|---|---|---|---|---|
| • | Battery Voltage | [V] | Device / Battery Voltage | date of measurement |
| • | Battery Reserve | [%] | Device / Battery Reserve | date of measurement |
| • | Atr. Pacing Impedance | [Ohm] | Leads / Pacing Impedance | date of measurement |
| • | Vtr. Pacing Impedance | [Ohm] | Leads / Pacing Impedance | date of measurement |
| • | Shock Impedance | [Ohm] | Leads / Shock Impedance | date of measurement |
| • | Atr. Sensing Amplitude | [mV] | Leads / Sensing Amplitude | date of measurement |
| • | Vtr. Sensing Amplitude | [Ohm] | Leads / Sensing Amplitude | date of measurement |
| | Event Ratios of | [%] | Antibrady. Pac. / Atrial Event Ratios | date of consecutive reports |
| • | Atrium-paced Ap and | | | |
| • | Atrium-sensed As | | | |
| | Event Ratios of | [%] | Antibrady. Pac. / Ventr. Event Ratios | date of consecutive reports |
| • | Atrium-paced Vp and | | | |
| • | Atrium-sensed Vs | | | |
| • | SVT Counter | - | Suprav. Epis. / SVT Counter | date of report |
| • | SVT Counter | - | Suprav. Epis. / SVT + VT Counter | date of report |
| • | VT1 Counter | - | Suprav. Epis. / SVT + VT Counter | date of report |
| • | VT2 Counter | - | Suprav. Epis. / SVT + VT Counter | date of report |
| • | VF Counter | - | Ventr. Epis. / Ventr. Counter | date of report |
| • | VT1 Counter | - | Ventr. Epis. / Ventr. Counter | date of report |
| • | VT2 Counter | - | Ventr. Epis. / Ventr. Counter | date of report |
| • | Ventr. ATP started | - | Ventr. Epis. / Ventr. ATP | date of report |
| • | Ventr. ATP successful | - | Ventr. Epis. / Ventr. ATP | date of report |
| • | Shocks started | - | Ventr. Epis. / Ventr. Shocks | date of report |
| • | Shocks canceled | - | Ventr. Epis. / Ventr. Shocks | date of report |
| • | Shocks successful | - | Ventr. Epis. / Ventr. Shocks | date of report |
| • | 30 J Shocks ineffective | - | Ventr. Epis. / Ventr. Shocks | date of report |

### Time Resolution

There are 4 different time resolutions for the graphs available:

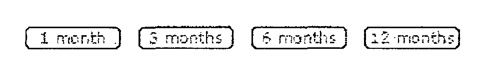

The selected period of time ends always with the last report date at the right side of the graph.

### Scrolling

→ The graphs are always displayed with the last report date at the right edge. It is intended to allow a half-page wise scrolling. However, this is currently not implemented in the prototype.

### 2.4.4.3 Device Status

This page displays the current state of the battery as well as of the device itself (see See Figure 2-10). The most current data is displayed in the table.

The trend of the battery voltage and the battery reserve can be reviewed in the graphs.

At the bottom the group status for the 'Device Status' group is displayed. It uses the group status indicator to display the current status of the group (see 2.4.2). If further details to a certain message are available, 'Details...' is displayed, which leads to an extra pop-up window with detailed information on the specific finding (see 2.4.4.8 for further explanations).

If any message of this group is still unacknowledged, the button 'Acknowledge' is displayed on the right side. Clicking on this button will only acknowledge the messages of the 'Device Status' group.

### See Figure 2-10. The Patient's Device Status Tab.

### 2.4.4.4 Leads

This page displays the most important values concerning the atrial and ventricular pacing lead as well as the ventricular shock lead (see See Figure 2-11). The most recent values are displayed in the table with their respective date of measurements.

The trend of the impedance values and the sensing amplitudes can be reviewed in the graphs.

At the bottom the group status for the 'Leads' group is displayed. It uses the group status indicator to display the current status of the group (see 2.4.2). If further details to a certain message are available, 'Details...' is displayed, which leads to an extra pop-up window with detailed information on the specific finding (see 2.4.4.8 for further explanations).

If any message of this group is still unacknowledged, the button 'Acknowledge' is displayed on the right side. Clicking on this button will only acknowledge the messages of the 'Leads' group.

### See Figure 2-11. The Patient's Leads Status Tab.

### 2.4.4.5 Antibradycardiac Pacing

This page displays the pace and sense event counters (See Figure 2-12). The table usually displays the event percentages for the time period since the last report and for the time period since last follow-up resp. since last counter restart. If a reference for calculation of these values is missing, '--' is displayed.

The trend of the daily measurements can be accessed via the graphs. Unlike the other graphs a vertical bar chart is used here for a better outlining of the paced and sensed events ratios.

At the bottom the group status for the 'Antibradycardiac Pacing' group is displayed. It uses the group status indicator to display the current status of the group (see 2.4.2). If further details to a certain message are available, 'Details...' is displayed, which leads to an extra pop-up window with detailed information on the specific finding (see 2.4.4.8 for further explanations).

If any message of this group is still unacknowledged, the button 'Acknowledge' is displayed on the right side. Clicking on this button will only acknowledge the messages of the 'Antibradycardiac Pacing' group.

### See Figure 2-12. The Patient's Antibradycardiac Pacing Tab.

The Belos DR-T currently does not transmit additional counter data. These could be found on this page otherwise.

### 2.4.4.6 Supraventricular Episodes

This page displays the SVT counter (See Figure 2-13). The table usually displays the new events percentages for the time period since the last report, for the time period since last follow-up and since implant resp. since last counter restart. If a reference for calculation of these values is missing, '--' is displayed.

The trend of the absolute counter values can be reviewed in the graphs.

At the bottom the group status for the 'Supraventricular Episodes' group is displayed. It uses the group status indicator to display the current status of the group (see 2.4.2). If further details to a certain message are available, 'Details...' is displayed, which leads to an extra pop-up window with detailed information on the specific finding (see 2.4.4.8 for further explanations).

If any message of this group is still unacknowledged, the button 'Acknowledge' is displayed on the right side. Clicking on this button will only acknowledge the messages of the 'Supraventricular Episodes' group.

### See Figure 2-13. The Patient's Supraventricular Episodes Tab.

If an implant would transmit additional data on supraventricular episodes, these could be found on this page.

### 2.4.4.7 Ventricular Episodes

This page displays the detection and therapy counters (see See Figure 2-14). These are divided into three aspects:
- Detection counter: VT1 counter, VT2 counter and VF counter
- ATP counter: started and successful
- Shock counter: started, canceled, successful, <30J shock ineffective and 30 J ineffective.

The table usually displays the new event counters for the time period since the last report, for the time period since last follow-up and since implant respectively since last counter restart. If a reference for calculation of these values is missing, '--' is displayed.

The trend of the absolute counters can be reviewed in the graphs.

### See Figure 2-14. The Patient's Ventricular Episodes Tab.

At the bottom the group status for the 'Ventricular Episodes' group is displayed. It uses the group status indicator to display the current status of the group (see 2.4.2). If further details to a certain message are available, 'Details...' is displayed, which leads to an extra pop-up window with detailed information on the specific finding (see 2.4.4.8 for further explanations).
If any message of this group is still unacknowledged, the button 'Acknowledge' is displayed on the right side. Clicking on this button will only acknowledge the messages of the 'Ventricular Episodes' group.

### 2.4.4.8 How to get Diagnosis Details

The details for a detected assessment are shown in a separate window. This window is reached by clicking on the 'Details ...' link on any one of the medical data pages. This displays some more information about the reasons why this assessment has been detected. The pop-up window in See Figure 2-15 outlines an example that gives further details on the assessment 'ERI detected'.

The header of the pop-up window repeats the selected assessment. Below, the contributing findings are listed in chronological order.

### See Figure 2-15. Pop-up Window to get more detailed information on a given alarm or warning.

### 2.5 Archive

This page displays the history of alarms and activated warnings for the current patient. See Figure 2-16 shows an example page.

The date and time in the left column indicate the (simulation) time, at which the evaluation was performed.

The middle column indicates the general status found in that evaluation, using the general status indicator.

The right column displays all alarms and activated warnings of the respective evaluation. It remains empty if the evaluation signals ok.

This page serves for reviewing the development of the assessments over time. There is no additional functionality on this page.

### See Figure 2-16. The Patient's Archive Tab.

### 2.6 Configuration

This page allows to set the individual configuration settings of the currently displayed patient. These settings are divided into three categories:
- Alarms
- Warnings
- Special Characteristics

The configuration settings will take effect from the next evaluation on. They are preserved even if a reset is done on the simulation control center!

The configuration page can always be reached by clicking the item 'Configuration' in the service center menu bar on the left side of any patient data page.

### 2.6.1 Alarms

On this page all implemented alarms are displayed (See Figure 2-17). For the meaning and background of each alarm additional information can be obtained by clicking on the 'Info' link. Appendix 6.2.1 gives an overview of all alarms that are currently supported by the Jupiter Demonstrator.

Alarms cannot be deactivated.

The only possible configuration for alarms refers to the kind of push media: e-mail, fax or SMS. Since this is only a simulation without real push media, this input is ignored. The would-have-been notifications are displayed on the Simulation Control Page (see 2.1.2).

### See Figure 2-17. Configuring the Patient's Alarm Settings.

### 2.6.2 Warnings

On this page the implemented warnings are displayed according to their groups. When selecting a group in the left table, the associated warnings are displayed in the right hand table. See Figure 2-18 shows as an example how to configure the warnings for the group 'Device Status'.

Appendix 6.22 gives an overview of all warnings that are currently supported by the Jupiter Demonstrator.

Warnings can be configured in two respects (options):
- Activation (Act.)
   If a warning is activated, the system scans the incoming data according to the rules of this warning. When the rules are fulfilled the warning is considered "detected".
- Notification (Not.)
   If the notification of a warning is activated, the system will issue a push media message as soon as the warning is detected. The notification can only be activated if the warning is activated itself.

### See Figure 2-18. Configuring the Patient's Warning Settings.

The following symbols are used to display the state of the activation and notification. By clicking on the symbols the state can be changed.
Individual warnings use two symbols: 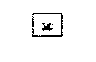 The option is deactivated for the warning. 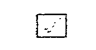 The option is activated for the warning.

For groups some warnings may be activated and some may not. Therefore an additional symbol is used: 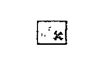 The group contains warnings with activated option as well as warnings with deactivated option.

Some warnings additionally allow to specify or select a cut-off value (e.g. see the list of limits for 'SMS Pending' in See Figure 2-18).
For each item on the configuration page additional information about its meaning can be obtained by clicking on the 'Info' link (see 2.6.4).
The push media for warnings can be set independently from the push media of alarms: e-mail, fax or SMS. But as with alarms the input is ignored. The would-have-been notifications are displayed on the Simulation Control Page (see 2.1.2).

The changes are not automatically stored within the system. To discard changes and restore the previous state to the screen, press the button 'Discard Changes'. The system's standard values can be restored to the screen via the button 'Standard Values'. In all cases, to store the changes permanently, press the button 'Save Changes' before selecting the next warning group or leaving the page.

### 2.6.3 Special Characteristics

The special characteristics allow to adapt several alarms and warnings to special characteristics of patient and device simultaneously without knowledge on the internal structure of the rule base. For the meaning of each special characteristics additional information can be obtained by clicking on the 'Info' link (see 2.6.4.).

Appendix 6.2.3 gives an overview of all special characteristics that are currently supported by the Jupiter Demonstrator.

Like with the warnings the changes are not automatically stored within the system. To discard changes and restore the previous state to the screen, press the button 'Discard Changes'. The system's standard values can be restored to the screen via the button 'Standard Values'. In all cases, to store the changes permanently, press the button 'Save Changes' before leaving the page.

### See Figure 2-19. Configuring the Patient's Special Characteristics.

### 2.6.4 How to get Information about Alarms and Warnings

The information about the meaning and background of a distinct assessment is shown in a separate window (see the example in See Figure 2-20). This pop-up window is reached by clicking on the 'Info' link on any one of the configuration pages. This displays some more information about the selected assessment.

### See Figure 2-20. Pop-up Window to get information on a special alarm or warning.

### 2.7 Help

Help texts are also shown in a separate window (e.g. see the Jupiter help page on 'Device Status' in See Figure 2-21). This window is reached by clicking on the 'Help' button on any page. Extracts of this manual can even be found on the help pages.

Each help page displays a context sensitive help text. Once the help window is open, other requested help pages will pop-up in the same window.

### See Figure 2-21. Pop-up Window to get help on a single web page.

### 2.8 Error Messages

During the testing phase, the Jupiter system has shown as rather stable and little error-prone. But as with any software, there can be given no warranty that no error at all will occur.

Most exceptional states are treated in the program so that the user will not notice them at all. Sometimes the program may display the Sign-in Page with a message describing what went wrong (e.g. in See Figure **2-22**). In these cases it is necessary and sufficient to sign in again. The most common reason for such a failure is a so-called session timeout. This simply means that the last user action was to long ago. The background for this is that the web server invalidates the access rights after a preset period of time to avoid unauthorized access. For the current installation the timeout interval is set to 30 minutes.

### See Figure 2-22: Example for Error Message 'Time-Out'

In some cases known errors have not been eliminated because this would have exceeded the limit of a prototypical simulator.

Almost always these errors are either caused by trivial reasons, which could be corrected very easily by the user. Or they are caused by technical reasons so difficult that the correction needs human action anyway. See Figure 2-23 shows how such errors might be displayed.

### See Figure 2-23: Example for Error Message 'An internal program error occurred'

Such a message can show up anywhere on a page even when the page is already half built up.

In most of that cases it will suffice to follow the advice and use the link to start 'from the beginning'.

Do not use the link to start 'from the beginning' if such an error message occurs in a help, details, or info page! This would display the Sign-in Page in this same window what can lead to unwanted behavior. Instead use the logout item on the menu of the data display window. This most probably will lead you to the Sign-in Page where an error message will be displayed.

If the new sign-in does not solve the problem please consult chapter 5.

### 3 Administration

After installation only a few administrational tasks are necessary: mainly to start and stop the demonstration or in case of troubles. All these tasks can be done via commands that are called by the icons in the desktop folder "Jupiter" (see See Figure 3-1).

### See Figure 3-1: Desktop Folder for Jupiter Administration Programs.

→ These tasks are needed only in the Demonstrator.
   In the real life system no such administrative tasks are done by the user.
   To better understand the more technical background of these tasks, you might want to consult chapter 4 first.

In general, you have to use only two simple commands: 'Start Jupiter' and 'Stop Jupiter'.

### 3.1 Start Jupiter

This starts the web server and opens a new browser window to display the Sign-in Page. In fact just 'Start Web Server' (see 3.5) and 'Start Browser' (see 3.4) are executed.

You use this to initialize your Jupiter session.

### 3.2 Stop Jupiter

This stops the web server and saves collected data that is used to analyze the program. You should have been told where to find these data and what to do with them after your study.

Please close your browser windows afterwards.

You use this after you have terminated your Jupiter session. The state of the session, especially the simulation date and all evaluations that are done so far, will be preserved.

### 3.3 Restart Jupiter

This stops and afterwards starts Jupiter again. In fact just 'Start Jupiter' (see 0) and afterwards 'Stop Jupiter' (see 3.2) are executed.

You should not need this command. Nevertheless, there might be some exceptional cases when you may use it to reset the runtime system. After the restart you need to sign in again.

### 3.4 Start Browser and Open Jupiter

This opens a new browser window to display the Sign-in Page. Browser windows that are already open keep unaffected. If some of theses windows display Jupiter pages, e.g. after 'Restart Jupiter', we recommend you to close them before.

You should not need this command. But nevertheless it is an easy way to return to Jupiter when you have opened other web pages.

### 3.5 Start the Web Server

This starts the web server. While the server is running a DOS window will be open. You will notice an DOS icon in the task menu bar.

You should not need this command. However in some exceptional cases, the web server may crash. Then the DOS window disappears and the browser either displays a message that it could not make contact or redirects you to an internet search page when you try to open a Jupiter page. In this case you need not to start all of Jupiter. Just restart the web server.

After the restart you need to sign in again.

Trying to start the web server when it already runs will do no harm. Just some error messages are shown, which you can safely ignore.

### 3.6 Stop the Web Server

This stops the web server. The DOS window that indicates that the server is running should disappear.

You should not need this command.

Trying to stop the web server when it does not run will do no harm. Just some error messages are shown, which you can safely ignore.

### 3.7 Logging Administration

In case of problems it is sometimes necessary to obtain information about the internal operations of the Jupiter system. Only for these cases and when asked for by a contact person, use the commands in the second Jupiter folder called 'Logging Administration'.

### • Switch on/off Logging

You should not switch on logging unless you are told so. It collects a lot of internal information and thus slows down the program significantly.

Caution: Be sure, that the server is down when using these commands!

### • Save Data, save Log Files

This saves and compresses the data files resp. the log files, which are created and processed during program execution. These files help to analyze possible program errors. In case of troubles the support may tell you to save the data or log files and to send it. You need not do this on your own.

Saving is done automatically when 'Stop Jupiter' is used. You should have been told where to find the saved files and what to do with them.

Caution: Be sure, that the server is down when using these commands!

### 4 Technical Background of the Demonstrator

To better understand the limitations of this prototype software and the reasons of possible errors, it is helpful to learn more about the technical background of this Demonstrator.

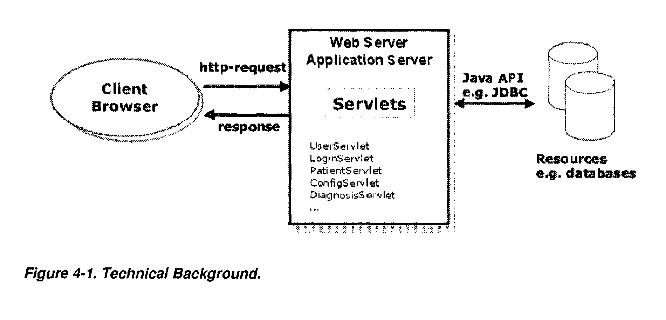

The Demonstrator in fact consists of several distinct programs (see See Figure 4-1):
- a browser (here Netscape Browser 7.1),
- a web server that includes several application programs (here Apache Tomcat Server 4.1.27),
- a database (here Microsoft Access Database) and
- as the most important part, a special diagnosis module that is added to the web server This program controls all the calculations for the evaluation of the raw Home Monitoring data.

### See Figure 4-1. Technical Background.

### Client Browser

This is the program that supplies the graphical interface to the user.
It receives data from the web server and displays it. In turn it accepts input from the user and delivers it back to the web server.
→ This is the only component that is needed in the intended real life system. For the Jupiter Demonstrator, it is strongly recommended to use Netscape Navigator 7.1 as your browser. Later on, any other standard browser should do as well.

### Web Server

This is the program that deals with the connection between the calculation module and the browser. Besides that, it provides the environment for the calculation module.
→ Because the Demonstrator is intended to run stand-alone, a local web server is necessary. On the real life system, this server runs at BIOTRONIK and the user will not notice it at all.

Caution: This web server may be accessible from other computers if the local computer is connected to a network. To avoid security risks, you should not run the web server when you are connected to the internet or another network with participants you do not trust.
In the Demonstrator Apache Tomcat/4.1.27 is used as web server.

### Database

This program provides the Home Monitoring data for the calculation. Furthermore it stores all data that must be made persistent. All the physicians' and patients' data are derived from real Home Monitoring data, but were made anonymous.
→ The Jupiter Demonstrator uses a local Microsoft Access database as an ODBC data source. On the real life system, a much more powerful database management system runs at BIOTRONIK and works as an 'online' system, i.e. it also handles incoming Home Monitoring data and controls a lot of concurrent accesses.

### Calculation Module

This is the newly developed part and the working horse of the whole system. Even so, it is not counted as a program of its own because it is tightly integrated within the web server. The user deals with this component only through inputs via the browser.
→ To simulate the Home Monitoring data day by day, the Jupiter Demonstrator uses a simulation time that can be controlled by the user (see 2.1.2). This incorporates an 'online' calculation of the newly imported Home Monitoring data. The calculation process is a bit time-consuming but, nevertheless, it is necessary to allow the simulation of the service center.
   The real life system would trigger those calculations shortly after the detection of transmitted Home Monitoring data and save the evaluated results in the database. Therefore the user would not notice them at all.

### 5 Trouble Shooting

This is a Demonstrator and not a real life system. So even if the Jupiter system has shown as rather stable and little error-prone during the testing phase, not all problems that may arise are handled. A failure in any of the four components or in the combination of two of them will lead to a failure of the whole system. Because this is almost always caused by installation errors, we do not handle most of them here and concentrate on problems the user might experience on a properly installed system.

The following topics give some hints on what to do if such a problem occurs.

### 5.1 What to do if no Help/Details/Info Pages show up?

By default, JavaScript should be enabled in your browser. Nevertheless, if mouse clicks on the 'Help' button or on the links 'Details' or 'Info' do not open a new window, it can be assumed that JavaScript is disabled. Therefore it is strongly recommended to enable JavaScript for the correct function of all the buttons and links.

With Netscape, please select 'Edit' and choose 'Preferences' from the top menu bar. In the new pop-up window select the category 'Advanced' >> 'Scripts & Plugins' at the left and activate the check-box 'Enable JavaScript for Navigator' at the right. Don't forget to press the OK button.

### 5.2 What to do if 'internal program errors' occur?

This is already mentioned in the chapter about error messages (see 2.8).

To remove this problem, just use the supplied link to start 'from the beginning'.

Caution: Do not use the link 'from the beginning' if such an error message occurs in a help, details, or info page! This would display the Sign-in Page in this same window what can lead to unwanted behavior. Instead, use the logout item of the menu bar on the data page. This most probably will lead you to the Sign-in Page (sic!) where an error message will be displayed.

### 5.3 What to do if there is no contact?

If there is no contact to the web server, Netscape usually triggers the Netscape Search, e.g. by a call to http://suche.netscape.delsuchelsearch.jsp?q=localhost.

Please try browsing to http://localhost:8080/hmsc/en_US/index.jsp. Press the Netscape Home Button for this if this link is configured as your start side.

If the problem still exists you have to restart Jupiter. Please consult 3.3 for more information on this.

### 5.4 What to do if nothing works to solve the problem?

If your problem persists please feel free to contact the service provider.

### 6 Appendix

### 6.1 Icons, Buttons, and Symbols

### 6.1.1 Status Indicators

### 6.1.1.1 General Status

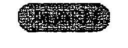

A serious problem, an alarm, detected. 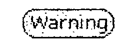 A less serious problem, a warning, found. 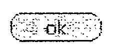 No abnormality detected (NAD). 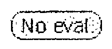 No status available. 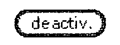 The patient is deactivated. The last status was Alarm, 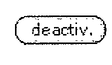 The patient is deactivated. The last status was Warning. 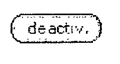 The patient is deactivated. The last status was NAD.

### 6.1.1.2 Group Status

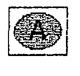

At least one alarm detected in this group. 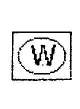 At least one activated warning detected in this group. This group has no alarms. 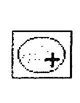 At least one deactivated important warning detected in this group. This group has no alarms or activated warnings. 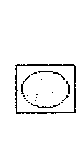 This group has no alarms nor activated warnings nor deactivated important warnings detected. The color is green. 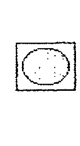 No evaluations available.
The color is grey.

### 6.1.1.3 Acknowledgement

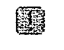

At least one alarm or activated warning has not yet been acknowledged in this patient. Reviewing the data and acknowledging the messages is recommended. 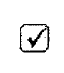 All detected alarms and activated warnings have been acknowledged in this patient.

### 6.1.2 Configuration Selection

### 6.1.2.1 Warnings

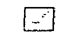

The warning will be displayed to the user if detected. 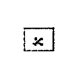 The warning will always be ignored and not displayed to the user.

### 6.1.2.2 Groups

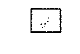

Any warning in the group will be displayed to the user if detected. 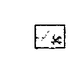 Some warnings in the group will be ignored and some are not. 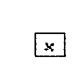 All warnings in the group will be ignored and not displayed to the user.

### 6.1.3 Symbols in Graphs

Symbols at the top of the graphs indicate counter resets or follow-ups that influence the values of parameters.
▼ A follow-up was detected in the corresponding message.
▼ A complete device reset was detected in the corresponding message.
▼ The content of the messages (SMS) may change depending on the firmware version of the device. This symbol signals that a change in the message content was detected in the corresponding message. It affects the internal interpretation of certain flags.
▼ The symbol signals a reset in event counters of sense and pace events. This may occur after reprogramming or together with a complete device reset.
▼ This symbol signals a reset of the tachy counter, e.g. together with a complete device reset. All episode and therapy counters are set to 0.

### 6.1.4 Buttons

### 6.1.4.1 Anywhere

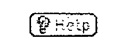

Displays a context sensitive help text within a separate window

### 6.1.4.2 Simulation Control

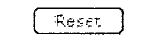

Resets the complete simulation to the start date, which is shown in the text. 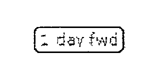 Advances the simulation by 1 day. All messages, which would have been received during this time, are evaluated. 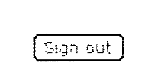 Retums to the Sign-In page. 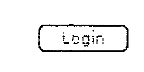 Enters the Jupiter pages.

### 6.1.4.3 Status Display

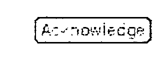

Acknowledges that the evaluation results for a special group are noticed. 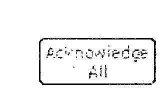 Acknowledges that the evaluation results for all groups are noticed.

### 6.1.4.4 Time Resolution for Graphs

Four different time spans can be selected as base for the parameter charts.

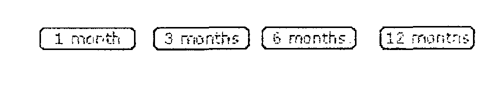

### 6.1.4.5 Configuration

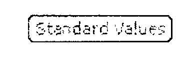

Resets the configuration to the predefined global values. 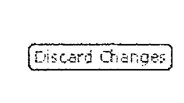 Resets the configuration to the settings that are previously saved for the selected patient. 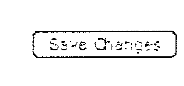 Saves the actual configuration.

### 6.2 Diagnostic Messages

The following list gives you an overview of possible alarms and warnings that may be detected by the Jupiter system for a Belos DR-T patient.

### 6.2.1 Alarms

### 6.2.1.1 Group Device Status

### Alarm: ERI Detected

| | |
|---|---|
| Description | This alarm appears as soon as the device signals that it has |
| | reached the ERI status. |
| Interpretation | ERI stands for Elective Replacement Indicator. This signals a low |
| | battery capacity, which allows only for a limited number of |
| | therapies. It is recommended to confirm this status by Follow-up |
| | and schedule a replacement in the upcoming weeks. |
| Recommended | For all patients. |
| Use | |
| Comment | None. |

### Alarm: SMS Pending and ERI

| | |
|---|---|
| Description | This alarm is issued if the center has not received any SMS from |
| | the patient for at least 7 days and the device signaled ERI state |
| | in the last received SMS. |
| Interpretation | The reasons for abrupt end of transmissions are manifold. |
| | Frequent reasons are: |
| | Patient device not turned on. |
| | Patient in vacation without notification. |
| | ··· |
| | However, in this special case, the device might have advanced: |
| | to the EOL after the last message. |
| Recommended | For all patients. |
| Use | |
| Comment | None. |

### Alarm: SMS Pending

| | |
|---|---|
| Description | This alarm is issued if the center has not received any SMS from |
| | the patient for at least 28 days. |
| Interpretation | The reasons for abrupt end of transmissions are manifold. |
| | Frequent reasons are: |
| | Patient device not turned on. |
| | Patient in vacation without notification. |
| | ... |
| Recommended | For all patients. |
| Use | |
| Comment | None. |

### Alarm: Reset Detected without FU

| | |
|---|---|
| Description | This alarm signals that a device reset took place outside a follow-up. |
| Interpretation | A reset, which is not initiated during a follow-up, is a rare event. It |
| | might be induced for example by strong electromagnetic |
| | interference. This reset might alter the programmed parameters, |
| | e.g. by restoring default parameters. Therefore, an early follow- |
| | up is strongly recommended. |
| Recommended | For all patients. |
| Use | |
| Comment | None. |

### Alarm: Special Device Status

| | |
|---|---|
| Description | This alarm is triggered by special device conditions and will be |
| | displayed as soon as the message is received in the center. |
| Interpretation | A special device status requires always an device interrogation, |
| | to allow further specification and resolution of the problem. |
| Recommended | For all patients. |
| Use | |
| Comment | None. |

### Alarm: Ventricular Detection Deactivated

| | |
|---|---|
| Description | This alarm is issued if the device signals that the ventricular |
| | tachycardia detection is deactivated. |
| Interpretation | Deactivated ventricular tachycardia detection signifies that the |
| | patient does not receive any therapy in case of life threatening |
| | arrhythmias at all. This should only be the case if the patient is |
| | under supervision with continuous cardiac monitoring and the |
| Recommended | possibility for external defibrillation. For all patients. |
| Use | |
| Comment | None. |

### Alarm: Ventricular Therapy Deactivated

| | |
|---|---|
| Description | This alarm is issued if the device signals that the ventricular |
| | tachycardia therapy is deactivated. |
| Interpretation | A deactivated ventricular tachycardia therapy signifies that the |
| | patient does not receive any therapy in case of life threatening |
| | arrhythmias at all. This should only be the case if the patient is: |
| | under supervision with continuous cardiac monitoring and the |
| | possibility for external defibrillation. |
| Recommended | For all patients. |
| Use | |
| Comment | None. |

### 6.2.1.2 Group Lead Failure

### Alarm: Atrial Pacing Lead

| | | | |
|---|---|---|---|
| Description | This alarm is issued if the atrial pacing lead measurements are | | |
| | severely deviating from their normal values. This alarm checks | | |
| | only the impedance, since the sensing amplitude and pacing | | |
| | threshold are only measured during foflow-ups. | | |
| Interpretation | This alarm is issued if at least one of the following findings are | | |
| | detected: | | |
| | The impedance has reached the maximum measurable value | | |
| | (above 3000 Ohm). | | |
| | The impedance has reached the minimum measurable value | | |
| | (below 200 Ohm). | | |
| | The impedance is above the upper limit (see table below) and | | |
| | rising further at a rate greater than 100 Ohm/week. | | |
| | The impedance is below the lower limit (see table below) and | | |
| | falling further at a rate greater than 100 Ohm/week. | | |
| | Since the normal values of the lead impedances might greatly | | |
| | differ between two electrodes, the electrodes are grouped into | | |
| | three different impedance groups: | | |
| | Group | Lower Limit | Upper Limit |
| | high | 1000 Ohm | 2600 Ohm |
| | normal | 350 Ohm | 2000 Ohm |
| | low | no lower limit | 1500 Ohm |
| | The group membership of the lead could be specified at "Special | | |
| | Characteristic" of the "Configuration" tab. | | |
| Recommended | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Alarm: Ventricular Pacing Lead

| | | | |
|---|---|---|---|
| Description | This alarm is issued if the ventricular pacing lead measurements | | |
| | are severely deviating from their normal values. This alarm | | |
| | checks only the impedance, since the sensing amplitude and | | |
| | pacing threshold are only measured during follow-ups. | | |
| | The impedance has reached the minimum measurable value | | |
| | (below 200 Ohm). | | |
| | The impedance is above the upper limit (see table below) and | | |
| | rising further at a rate greater than 100 Ohm/week. | | |
| | The impedance is below the lower limit (see table below) and | | |
| | falling further at a rate greater than 100 Ohm/week. | | |
| | Since the normal values of the lead impedances might greatly | | |
| | differ between two electrodes, the electrodes are grouped into | | |
| | three different impedance groups: | | |
| | Group | Lower Limit | Upper Limit |
| | high | 1000 Ohm | 2600 Ohm |
| | normal | 350 Ohm | 2000 Ohm |
| | low | no lower limit | 1500 Ohm |
| | The group membership of the lead could be specified at "Special | | |
| | Characteristic" of the "Configuration" tab. | | |
| Recommended | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Alarm: Ventricular Shock Lead

| | |
|---|---|
| Description | This alarm signals that the impedance of the ventricular shock |
| | electrode displays severely deviated values. |
| Interpretation | This alarm is issued if at least one of the following findings are |
| | detected: |
| | The impedance has reached the maximum measurable value |
| | (above 150 Ohm). |
| | The impedance has reached the minimum measurable value |
| | (below 25 Ohm) |
| | The impedance is 110 Ohm or above and rising 5 Ohm or more |
| | regarding the last three transmitted shock impedance |
| | measurements. |
| | The impedance is 30 Ohm or below and falling 5 Ohm or more |
| | regarding the last three transmitted shock impedance |
| | measurements. |
| Recommended | For all patients. |
| Use | |
| Comment | None. |

### 6.2.1.3 Group Ventricular Therapies

### Alarm: Some Ventricular Maximum Energy Shocks since Follow-up

| | |
|---|---|
| Description | This alarm signals that the counter for ineffective ventricular |
| | maximum energy shocks has been incremented at least by 5 |
| | since the last reported follow-up. This alarm takes only episodes |
| | into account, which are not mentioned in the same report as a |
| | Follow-up. |
| Interpretation | This alarms reports that several ineffective ventricular maximum |
| | energy shocks have occurred since last follow-up. Once |
| | acknowledged, it will not reappear before a new follow-up is |
| | detected in the received messages. A high number of ineffective |
| | maximum energy shocks usually points at inadequate therapies |
| | for SVT, incessant ventricular tachycardia or lead problems |
| | including rise of defibrillation threshold. |
| Recommended | For all patients. |
| Use | |
| Comment | None. |

### 6.2.2 Warnings

### 6.2.2.1 Group Device Status

| Warning: Battery Status Outdated | |
|---|---|
| Description | This warning is activated, if the device reports either MOL1 and |
| | at least 20 started shocks or MOL2 and at least 8 started shocks |
| Interpretation | The automatic measurement of the battery voltage is suspended |
| | for 24 hours after charging of the capacitors to avoid false low |
| Recommended | For all patients. |
| Use | |
| Use | |
| Comment | None. |

### Warning: First Message Received

| | |
|---|---|
| Description | This warning is issued if the very first message from the device is |
| | received in the service center. |
| Interpretation | The first message should always be checked manually, since |
| | many of the surveillance rules are triggered only by changes in |
| | parameters not by absolute values. In addition this is a good: |
| | opportunity to check the activated warnings for the patient. |
| | This warning will not be issued, if it is the first message after a |
| | longer pause. |
| Recommended | For all patients. |
| Use | |
| Comment | None. |

### Warning: Reset Detected with FU

| | |
|---|---|
| Description | This warning is issued if at least one device reset and a follow-up |
| | are reported in the same message by the device. |
| Interpretation | Due to the limited time resolution of the system it can not be |
| | distinguished whether the follow-up took place before, during or |
| | after the follow-up. The likelihood of a second spontaneous |
| | device reset is minimal, if a device reset has been performed |
| | during the follow-up. |
| Recommended | For all patients. |
| Use | |
| Comment | None. |

### Warning: SMS Pending

| | |
|---|---|
| Description | This warning is issued if the center has not received any SMS |
| | from the patient for a certain number of days. This number is |
| | either 3, 7 or 14 days and can be chosen by the user on the |
| | configuration page for warnings, group device status. The |
| | default value is 3. |
| Interpretation | The reasons for abrupt end of transmissions are manifold. |
| | Frequent reasons are: |
| | Patient device not turned on. |
| | Patient in vacation without notification. |
| | ··· |
| Recommended | For all patients. |
| Use | |
| Comment | None. |

### Warning: Special Device Status

| | |
|---|---|
| Description | This warning is issued if any special device status was detected, |
| | but it is currently resolved. |
| Interpretation | This message follows the corresponding alarm "Special Device |
| | Status", if the alarm has not been acknowledged, but the special |
| | device status was resolved. Since the status may only be resets |
| | by interrogation it can be assumed that the device is currently |
| | proper functioning. However, the special device status is an |
| | important event, for which the homemonitoring physician should |
| | be notified, even if the problem is currently resolved. Note: A |
| | special device status requires always an device interrogation, to |
| | allow further specification and resolvement of the problem. |
| Recommended | For all patients. |
| Use | |
| Comment | None. |

### 6.2.2.2 Group Leads

### Warning: Atrial Pacing Lead

| | | | |
|---|---|---|---|
| Description | This warning is issued if the atrial pacing lead measurements | | |
| | display unusual values. This warning checks only the | | |
| | impedance, since the sensing amplitude and pacing threshold | | |
| | are only measured during follow-ups. | | |
| Interpretation | This warning is issued if at least one of the following findings are | | |
| | detected: | | |
| | The impedance is above the upper limit (see table below). | | |
| | The impedance is below the lower limit (see table below). | | |
| | The impedance is rising at a rate greater than 200 Ohm/week. | | |
| | The impedance is falling at a rate greater than 200 Ohm/week. | | |
| | Since the normal values of the lead impedances might greatly | | |
| | differ between two electrodes, the electrodes are grouped into | | |
| | three different impedance groups: | | |
| | Group | Lower Limit | Upper Limit |
| | high | 1000 Ohm | 2600 Ohm |
| | normal | 350 Ohm | 2000 Ohm |
| | low | no lower limit | 1500 Ohm |
| | The group membership of the lead could be specified at "Special | | |
| | Characteristic" of the "Configuration" tab. | | |
| Recommended, | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Warning: Ventricular Pacing Lead

| | | | |
|---|---|---|---|
| Description | This warning is issued if the ventricular pacing lead | | |
| | measurements display unusual values. This warning checks only | | |
| | the impedance, since the sensing amplitude and pacing | | |
| | threshold are only measured during follow-ups. | | |
| Interpretation | This warning is issued if af least one of the following findings are | | |
| | detected: | | |
| | The impedance is above the upper limit (see table below). | | |
| | The impedance is below the lower limit (see table below). | | |
| | The impedance is rising at a rate greater than 200 Ohm/week. | | |
| | The impedance is falling at a rate greater than 200 Ohm/week. | | |
| | Since the normal values of the lead impedances might greatly | | |
| | differ between two electrodes, the electrodes are grouped into | | |
| | three different impedance groups: | | |
| | Group | Lower Limit | Upper Limit |
| | high | 1000 Ohm | 2600 Ohm |
| | normal | 350 Ohm | 2000 Ohm |
| | low | no lower limit | 1500 Ohm |
| | The group membership of the lead could be specified at "Special | | |
| | Characteristic" of the "Configuration" tab. | | |
| Recommended | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Warning: Ventricular Shock Lead

| | |
|---|---|
| Description | This warning signals that the impedance of the ventricular shock |
| | electrode displays unusual values. |
| Interpretation | This warning is issued if at least one of the following findings are |
| | detected: |
| | The impedance is above 110 Ohm. |
| | The impedance is below 30 Ohm. |
| | The impedance is rising 10 Ohm or more regarding the last three |
| | transmitted shock impedance measurements. |
| | The impedance is falling 10 Ohm or more regarding the last |
| | three transmitted shock impedance measurements. |
| Recommended | For all patients. |
| Use | |
| Comment | None. |

### 6.2.2.3 Group Antibradycardiac Pacing

### Warning: High Ventricular Pace Ratio

| | |
|---|---|
| Description | This warning is issued if the percentage of paced ventricular |
| | events is above a certain value. This value is either 50%, 60%, |
| | 70%, 80%, 90% or 95% and can be defined by the Home- |
| | Monitoring physician on the "Warning" page of the |
| | "Configuration" tab. |
| Interpretation | The ventricular pacing ratio values based on 24h measurements |
| | are scanned for limit violations. |
| Recommended | This warning is deactivated by default, but may be useful in |
| Use | patients, in which ventricular pacing has deteriorating effects, |
| | e.g. right ventricular pacing in chronic heart failure patients may |
| | lead to acute decompensation. When activated, this warning will |
| | signal that the patient is predominantly paced and might be a |
| | candidate for reprogramming. |
| Comment | This warning is only useful in certain patients and will therefore |
| | not be displayed, if deactivated but detected. This behavior is in |
| | contrast to warnings with higher priority. |

### Warning: Low Atrial Pace Ratio

| | |
|---|---|
| Description | This warning is issued if the percentage of paced atrial events is |
| | below a certain value. This value is either 80%, 90% or 95% and |
| | can be defined by the Home-Monitoring physician on the |
| | "Warning" page of the "Configuration" tab. |
| Interpretation | The atrial pacing ratio values based on 24h measurements are |
| | scanned for limit violations. |
| Recommended | This warning is deactivated by default, but may be useful in |
| Use | patients in which atrial pacing has therapeutic effects, e.g. |
| | electric stabilization of the atrium for the prevention of |
| | arrhythmia. When activated, this warning will signal that the: |
| | patient is no longer predominantly paced and might be a |
| | candidate for reprogramming. |
| Comment | This warning is only useful in certain patients and will therefore |
| | not be displayed, if deactivated but detected. This behavior is in |
| | contrast to warnings with higher priority. |

### Warning: Low Atrial Sense Ratio

| | |
|---|---|
| Description | This warning is issued if the percentage of sensed atrial events is |
| | below a certain value. This value is either 80%, 90% or 95% and |
| | can be specified by the Home-Monitoring physician on the |
| | "Warning" page of the "Configuration" tab. |
| Interpretation | The atrial sense ratio values based on 24h measurements are |
| | scanned for limit violations. |
| Recommended | This warning is deactivated by default, but may be useful in |
| Use | patients in which atrial pacing leads to undesired effects, e.g. |
| Use | arrhythmia induction. |
| Comment | This warning is only useful in certain patients and will therefore |
| | not be displayed, if deactivated but detected. This behavior is in |
| | contrast to warnings with higher priority. |

### Warning: Low Ventricular Pace Ratio

| | |
|---|---|
| Description | This warning is issued if the percentage of paced ventricular |
| | events is below a certain value. This value is either 80%, 90% or |
| | 95% and can be defined by the Home-Monitoring physician on |
| | the "Warning" page of the "Configuration" tab. |
| Interpretation | The ventricular pacing ratio values based on 24h measurements |
| | are scanned for limit violations. |
| Recommended | This warning is deactivated by default, but may be useful in |
| Use | patients in which ventricular pacing has therapeutic effects, e.g.: |
| | - electric stabilization of the ventricle for the prevention of |
| | arrhythmia. |
| | - hemodynamic benefit in biventricular pacing of CHF patients |
| | - hemodynamic benefit in right ventricular apex pacing in patients |
| | with obstructive hypertrophic cardiomyopathy. |
| | When activated, this warning will signal that the patient is no |
| | longer predominantly paced and might be a candidate for |
| | reprogramming. |
| Comment | This warning is only useful in certain patients and will therefore |
| | not be displayed, if deactivated but detected. This behavior is in |
| | contrast to warnings with higher priority. |

### Warning: Low Ventricular Sense Ratio

| | |
|---|---|
| Description | This warning is issued if the percentage of sensed ventricular |
| | events is below a certain value. This value is either 80%, 90% or |
| | 95% and can be specified by the Home-Monitoring physician on |
| | the "Warning" page of the "Configuration" tab. |
| Interpretation | The ventricular sense ratio values based on 24h measurements |
| | are scanned for limit violations. |
| Recommended | This warning is deactivated by default, but may be useful in |
| Use | patients in which ventricular pacing leads to undesired effects, |
| | e.g. arrhythmia induction, hemodynamic worsening. |
| Comment | This warning is only useful in certain patients and will therefore |
| | not be displayed, if deactivated but detected. This behavior is in |
| | contrast to warnings with higher priority. |

### 6.2.2.4 Group Supraventricular Episodes

### Warning: SVT Episode Cluster

| | |
|---|---|
| Description | This warning signals that the SVT episode counter has been |
| | incremented at least by 40 over the last 48 hours. This warning |
| | takes only episodes into account, which are not mentioned in the |
| | same report as a Follow-up. |
| Interpretation | This warning reports that a certain number of episodes have |
| | accumulated during the last 48 hours. This warning uses a |
| | sliding window and - once acknowledged - will remain visible |
| | until the number of episodes in 48 hours is below the limit again. |
| Recommended | For all patients. |
| Use | |
| Comment | None. |

### Warning: SVT Episode with Follow-up

| | |
|---|---|
| Description | This warning signals an increment of the episode counter for the |
| | SVT, which is reported in the same message as a follow-up. |
| Interpretation | Due to the limited time resolution it cannot be decided, whether |
| | the episodes occurred before, during or after the follow-up. Only |
| | by consulting the follow-up data it can be determined if all |
| | episodes have been reviewed by a physician. |
| Recommended | For all patients. |
| Use | |
| Comment | None. |

### 6.2.2.5 Group Ventricular Episodes / Detection

### Warning: First VT-1 Episode since Follow-up

| | |
|---|---|
| Description | This warning signals that the episode counter of the VT-1 zone |
| | has been incremented since the last follow-up. This warning |
| | same report as a Follow-up. |
| Interpretation | This warning reports that the first episode or episodes since |
| | follow-up have occurred. Once acknowledged, it will not |
| | reappear before a new follow-up is detected in the received |
| | messages. |
| Recommended | For all patients, especially in the early phase after ICD implant. |
| Use | In the chronic phase, this warning may be deactivated, if not |
| | every episode of the patient requires intervention by the |
| | physician. |
| Comment | None. |

### Warning: First VT-2 Episode since Follow-up

| | |
|---|---|
| Description | This warning signals that the episode counter of the VT-2 zone |
| | has been incremented since the last follow-up. This warning |
| | takes only episodes into account, which are not mentioned in the |
| | same report as a Follow-up. |
| Interpretation | This warning reports that the first episode or episodes since |
| | follow-up have occurred. Once acknowledged, it will not |
| | reappear before a new follow-up is detected in the received |
| | messages. |
| Recommended | For all patients, especially in the early phase after ICD implant. In |
| Use | the chronic phase, this warning may be deactivated, if not every |
| | episode of the patient requires intervention by the physician. |
| Comment | None. |

### Warning: First VF Episode since Follow-up

| | |
|---|---|
| Description | This warning signals that the episode counter of the VF zone has |
| | been incremented since the last follow-up. This warning takes |
| | only episodes into account, which are not mentioned in the same |
| | report as a Follow-up. |
| Interpretation | This warning reports that the first episode or episodes since |
| | follow-up have occurred. Once acknowledged, it will not |
| | reappear before a new follow-up is detected in the received |
| | messages. |
| Recommended | For all patients, especially in the early phase after ICD implant. In |
| Use | the chronic phase, this warning may be deactivated, if not every |
| | episode of the patient requires intervention by the physician. |
| Comment | None. |

### Warning: New VF Episode

| | |
|---|---|
| Description | This warning signals that the episode counter of the VF zone has |
| | been incremented since the last report. This warning takes only |
| | episodes into account, which are not mentioned in the same |
| | report as a Follow-up. |
| Interpretation | This warning reports any new VF episode or episodes. |
| Recommended | For all patients, especially in the early phase after ICD implant. In |
| Use | the chronic phase, this warning may be deactivated, if not every |
| | episode of the patient requires intervention by the physician. |
| Comment | None. |

### Warning: VF Episode Accumulation

| | |
|---|---|
| Description | This warning signals that the episode counter of the VF zone has |
| | been incremented at least by 3 over the last 4 weeks. This |
| | warning takes only episodes into account, which are not |
| | mentioned in the same report as a Follow-up. |
| Interpretation | This warning reports that a certain number of episodes have |
| | accumulated during the last 4 weeks. This warning uses a sliding |
| | window and - once acknowledged - will remain visible until the |
| | number of episodes in 4 weeks is below the limit again. |
| Recommended | For all patients, especially if gradual worsening should be |
| Use | detected. |
| Comment | None. |

### Warning: VF Episode Cluster

| | |
|---|---|
| Description | This warning signals that the episode counter of the VF zone |
| | has been incremented at least by 3 over the last 48 hours. This |
| | warning takes only episodes into account, which are not |
| | mentioned in the same report as a Follow-up. |
| Interpretation | This warning reports that a certain number of episodes have |
| | accumulated during the last 48 hours. This warning uses a |
| | sliding window and - once acknowledged - will remain visible |
| | until the number of episodes in 48 hours is below the limit again. |
| Recommended | For all patients. |
| Use | |
| Comment | None. |

### Warning: VF Episode with Follow-up

| | |
|---|---|
| Description | This warning signals an increment of the episode counter for the |
| | VF zone, which is reported in the same message as a follow-up. |
| Interpretation | Due to the limited time resolution it cannot be decided, whether |
| | the episodes occurred before, during or after the follow-up. Only |
| | by consulting the follow-up data it can be determined if all |
| | episodes have been reviewed by a physician. |
| Recommended | For all patients. |
| Use | |
| Comment | None. |

### Warning: VT-1 Episode Accumulation

| | |
|---|---|
| Description | This warning signals that the episode counter of the VT-1 zone |
| | has been incremented at least by 15 over the last 4 weeks. This |
| | warning takes only episodes into account, which are not |
| | mentioned in the same report as a Follow-up. |
| Interpretation | This warning reports that a certain number of episodes have |
| | accumulated during the last 4 weeks. This warning uses a sliding |
| | window and - once acknowledged - will remain visible until the |
| | number of episodes in 4 weeks is below the limit again. |
| Recommended | For all patients, especially if gradual worsening should be |
| Use | detected. |

### Warning: VT-1 Episode Cluster

| | |
|---|---|
| Description | This warning signals that the episode counter of the VT-1 zone |
| | has been incremented at least by 10 over the last 48 hours. This |
| | warning takes only episodes into account, which are not |
| | mentioned in the same report as a Follow-up. |
| Interpretation | This warning reports that a certain number of episodes have |
| | accumulated during the last 48 hours. This warning uses a |
| | sliding window and - once acknowledged - will remain visible |
| | until the number of episodes in 48 hours is below the limit again. |
| Recommended | For all patients. |
| Use | |
| Comment | None. |

### Warning: VT-1 Episode with Follow-up

| | |
|---|---|
| Description | This warning signals an increment of the episode counter for the |
| | VT-1 zone, which is reported in the same message as a follow- |
| | up. |
| Interpretation | Due to the limited time resolution it can not be decided, whether |
| | the episodes occurred before, during or after the follow-up. Only |
| | by consulting the follow-up data it can be determined if all |
| | episodes have been reviewed by a physician. |
| Recommended | For all patients. |
| Use | |
| Use | |
| Comment | None. |

### Warning: VT-2 Episode Accumulation

| | |
|---|---|
| Description | This warning signals that the episode counter of the VT-2 zone |
| | has been incremented at least by 10 over the last 4 weeks. This |
| | warning takes only episodes into account, which are not |
| | mentioned in the same report as a Follow-up. |
| Interpretation | This warning reports that a certain number of episodes have |
| | accumulated during the last 4 weeks. This warning uses a sliding |
| | window and - once acknowledged - will remain visible until the |
| | number of episodes in 4 weeks is below the limit again. |
| Recommended | For all patients, especially if gradual worsening should be |
| Use | detected. |
| Comment | None. |

### Warning: VT-2 Episode Cluster

| | |
|---|---|
| Description | This warning signals that the episode counter of the VT-2 zone |
| | has been incremented at least by 5 over the last 48 hours. This |
| | warning takes only episodes into account, which are not |
| | mentioned in the same report as a Follow-up. |
| Interpretation | This warning reports that a certain number of episodes have |
| | accumulated during the last 48 hours. This warning uses a |
| | sliding window and - once acknowledged - will remain visible |
| | until the number of episodes in 48 hours is below the limit again. |
| Recommended | For all patients. |
| Use | |
| Comment | None. |

### Warning: VT-2 Episode with Follow-up

| | |
|---|---|
| Description | This warning signals an increment of the episode counter for the |
| | VT-2 zone, which is reported in the same message as a follow-up. |
| Interpretation | Due to the limited time resolution it can not be decided, whether |
| | the episodes occurred before, during or after the follow-up. Only |
| | by consulting the follow-up data it can be determined if all |
| | episodes have been reviewed by a physician. |
| Recommended | For all patients. |
| Use | |
| Comment | None. |

### 6.2.2.6 Group Ventricular Episodes / Therapy

### Warning: Accumulation of Delivered Ventricular Shocks

| | |
|---|---|
| Description | This warning signals that the counter of the delivered ventricular |
| | shocks has been incremented at least by 5 over the last 4 |
| | weeks. This warning takes only episodes into account, which are |
| | not mentioned in the same report as a Follow-up. |
| Interpretation | This warning reports that a certain number of delivered |
| | ventricular shocks have accumulated during the last 4 weeks. |
| | This warning uses a sliding window and - once acknowledged - |
| | will remain visible until the number of delivered ventricular |
| | shocks in 4 weeks is below the limit again. |
| Recommended | For all patients. |
| Use | |
| Comment | None. |

### Warning: Cluster of Delivered Ventricular Shocks

| | |
|---|---|
| Description | This warning signals that the counter of the delivered ventricular |
| | shocks has been incremented at least by 3 over the last 48 |
| | hours. This warning takes only episodes into account, which are |
| | not mentioned in the same report as a Follow-up. |
| Interpretation | This warning reports that a certain number of delivered |
| | ventricular shocks have accumulated during the last 48 hours. |
| | This warning uses a sliding window and - once acknowledged - |
| | will remain visible until the number of delivered ventricular |
| | shocks in 48 hours is below the limit again. |
| Recommended | For all patients. |
| Use | |
| Comment | None. |

### Warning: Delivered Ventricular Shocks with Follow-up

| | |
|---|---|
| Description | This warning signals an increment of the counter of the delivered |
| | ventricular shocks, which is reported in the same message as a |
| | follow-up. |
| Interpretation | Due to the limited time resolution it can not be decided, whether |
| | the shocks occurred before, during or after the follow-up. Only by |
| | consulting the follow-up data it can be determined if all shocks |
| | have been reviewed by a physician. |
| Recommended | For all patients. |
| Use | |
| Comment | None. |

### Warning: High Ratio of Ineffective Ventricular ATP since Follow-up

| | |
|---|---|
| Description | This warning signals that the ratio of the ineffective to started |
| | ventricular ATP sequences is above 80%. This ratio is only |
| | evaluated for the ATP sequences, which have occurred after the |
| | fast follow-up. A minimum of 20 started ATP is required for this |
| | evaluation. This warning takes only sequences into account, |
| | which are not mentioned in the same report as a Follow-up. |
| Interpretation | This warning reports that a certain percentage of ventricular ATP |
| | sequences is ineffective. The time frame is the period since the |
| | last follow-up. A high number of ineffective ATP usually points at |
| | inadequate therapies, changed properties of the ventricular |
| | tachycardia or lead problems with oversensing. |
| Recommended | For all patients. |
| Use | |
| Use | |
| Comment | None. |

### Warning: New Ventricular Ineffective Maximum Energy Shock

| | |
|---|---|
| Description | This warning signals that the counter for ineffective ventricular |
| | maximum energy shocks has been incremented since the last |
| | report. This warning takes only episodes into account, which are |
| | not mentioned in the same report as a Follow-up. |
| Interpretation | This warning reports any ineffective ventricular maximum energy |
| | shock or shocks. An ineffective maximum energy shock usually |
| | points at inadequate therapies for SVT or lead problems |
| | including rise of defibrillation threshold. |
| Recommended | For all patients. |
| Use | |
| Comment | None. |

### Warning: Some Canceled Shocks since Follow-up

| | |
|---|---|
| Description | This warning signals that the counter of the canceled ventricular |
| | shocks has been incremented at least by 5 since the last |
| | reported follow-up. This warning takes only episodes into |
| | account, which are not mentioned in the same report as a |
| | Follow-up. |
| Interpretation | This warning reports that a certain number of cancelled |
| | ventricular shocks have occurred since last follow-up. Once |
| | acknowledged, it will not reappear before a new follow-up is |
| | detected in the received messages. A high number of canceled |
| | shocks usually points at non-sustained tachycardia, for which the |
| | detection phase should be prolonged, or at lead problems with |
| | consecutive over-sensing. |
| Recommended | For all patients. |
| Use | |
| Comment | None. |

### Warning: Some Canceled Shocks with Follow-up

| | |
|---|---|
| Description | This warning signals an increment of the counter for canceled |
| | ventricular shocks by at least 5. This is reported in the same |
| | message as a follow-up. |
| Interpretation | Due to the limited time resolution it cannot be decided, whether |
| | the shocks occurred before, during or after the follow-up. Only by |
| | consulting the follow-up data it can be determined if all shocks |
| | have been reviewed by a physician. |
| Recommended | For all patients. |
| Use | |
| Comment | None. |

### Warning: Some Ventricular Ineffective Non-maximum Energy Shocks since Follow-up

| | |
|---|---|
| Description | This warning signals that the counter for ineffective ventricular |
| | non-maximum energy shocks has been incremented at least by |
| | 5 since the last reported follow-up. This warning takes only |
| | episodes into account, which are not mentioned in the same |
| | report as a Follow-up. |
| Interpretation | This warning reports that a certain number of ineffective |
| | ventricular non-maximum energy shocks have occurred since |
| | last follow-up. Once acknowledged, it will not reappear before a |
| | new follow-up is detected in the received messages. A high |
| | number of ineffective non-maximum energy shocks usually |
| | points at a programmed value very near to the true defibrillation |
| | threshold, use of low-energy cardioversion shocks or a rise of |
| | defibrillation threshold. |
| Recommended | For all patients. |
| Use | |
| Comment | None. |

### Warning: Some Ventricular Ineffective Non-maximum Energy Shocks with Follow-up

| | |
|---|---|
| Description | This warning signals an increment of the counter for ineffective |
| | ventricular non-maximum energy shocks by at least 5. This is |
| | reported in the same message as a follow-up. |
| Interpretation | Due to the limited time resolution it can not be decided, whether |
| | the shocks occurred before, during or after the follow-up. Only by |
| | consulting the follow-up data it can be determined if all shocks |
| | have been reviewed by a physician. |
| Recommended | For all patients. |
| Use | |
| Comment | None. |

### Warning: Ventricular Ineffective Maximum Energy Shock with Follow-up

| | |
|---|---|
| Description | This warning signals an increment of the counter of the |
| | ineffective ventricular maximum energy shocks, which is reported |
| | in the same message as a follow-up. |
| Interpretation | Due to the limited time resolution it can not be decided, whether |
| | the shocks occurred before, during or after the follow-up. Only by |
| | consulting the follow-up data it can be determined if all shocks |
| | have been reviewed by a physician. |
| Recommended | For all patients. |
| Use | |
| Comment | None. |

### 6.2.3 Special Characteristics

### 6.2.3.1 Group Leads

### Special Characteristic: Ventricular Lead Pacing Impedance

| | | | |
|---|---|---|---|
| Description | This special characteristic influences the alarms and warnings for | | |
| | the ventricular lead. | | |
| Interpretation | Different electrodes require different normal ranges of the pacing | | |
| | impedance. In addition to the common electrode ("normal") the | | |
| | systems allows to select electrodes with very high or very low | | |
| | impedance values, which are considered normal for the specific | | |
| | electrode. | | |
| | The electrodes are grouped into three different impedance groups: | | |
| | Group | Lower Limit | Upper Limit |
| | high | 1000 Ohm | 2600 Ohm |
| | normal | 400 Ohm | 2000 Ohm |
| | low | no lower limit | 1500 Ohm |
| | The group membership of the lead can be specified at "Special | | |
| | Characteristic" of the "Configuration" tab. | | |
| Recommended | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Special Characteristic: Atrial Lead Pacing Impedance

| | | | |
|---|---|---|---|
| Description | This special characteristic influences the alarms and warnings for | | |
| | the atrial lead.. | | |
| Interpretation | Different electrodes require different normal ranges of the pacing | | |
| | impedance. In addition to the common electrode ("normal") the | | |
| | systems allows to select electrodes with very high or very low | | |
| | impedance values, which are considered normal for the specific | | |
| | electrode. | | |
| | The electrodes are grouped into three different impedance groups: | | |
| | Group | Lower Limit | Upper Limit |
| | high | 1000 Ohm | 2600 Ohm |
| | normal | 400 Ohm | 2000 Ohm |
| | low | no lower limit | 1500 Ohm |
| | The group membership of the lead can be specified at "Special | | |
| | Characteristic" of the "Configuration" tab. | | |
| Recommended | For all patients. | | |
| Use | | | |
| Comment | The group "low" is usually used for pacing leads in which | | |
| | impedances around 300 and below are tolerated. These leads | | |
| | often display minimum impedance values without being broken. | | |
| | Therefore the range of normal values is open towards low | | |
| | values, ignoring any finding referring to "lower limit". | | |

### Special Characteristic: Atrial Lead State

| | | |
|---|---|---|
| Description | This special characteristic signals the functional state of the atrial lead. | |
| Interpretation | The atrial lead has three different states: | |
| | State | Meaning |
| | | All atrial values were evaluated by the respective |
| | on | |
| | | alarms and warnings. |
| | | This will inhibit the evaluation of the atrial values |
| | sensing | related to pacing in the respective alarms and |
| | only | |
| | | Warnings. |
| | off | This will inhibit the evaluation of all atrial values in the |
| | | respective alarms and warnings. |
| | This could be specified at "Special Characteristic" of the | |
| | "Configuration" tab. | |
| Recommended | All patients: | |
| Use | It is recommended to change this option to "off", if the ICD is in | |
| | VVI(R) mode, to "sensing only" if the ICD is in VDD(R) mode and | |
| | to "on" if the ICD is in DDD(R) mode. | |
| Comment | None. | |

## Claims

1. Data management system for processing of physiological or technical data or both to generate a display control signal,
with a data interface, which comprises
a patient data interface to receive data, which represent physiological parameter of a patient or technical parameter of a medical device, in particular of an implant like a cardiac pacemaker or a defibrillator, cardioverter or the like, and
a user interface to receive data to be entered by a user,
with a data base adapted to store data received via the patient data interface and to retrieve stored data, and
with a processing unit which is at least indirectly connected to the user interface, said processing unit is adapted to retrieve and to process data stored in the data base and to create a display control signal for a graphical data display such that the display control signal depends on said data,
**characterized in that**,
said processing unit is connected to a rule base and adapted to process said data according to rules stored in the rule base
said rule base comprises rules defining alarm states such that an alarm state is to be determined by the processing unit, if the data stored in the data base indicate an acute health disaster of a patient or an acute failure of a device,
wherein the processing unit is adapted to create a display control signal for displaying an alarm symbol dependent on a user identification signal received from the user interface such that for each patient or device being assigned to an identified user all actual alarms states are immediately displayed by an alarm symbol.

2. Data management system according to claim 1, wherein
the rule base comprises rules which define a warning state such that an warning state is to be determined by the processing unit if said data indicates an upcoming health complication or a minor technical failure, and
the processing unit is adapted to generate a display control signal for displaying for each patient or device being assigned to an identified user a warning symbol on a display, if for said patient or device a warning state as defined by the rule base is given by an actual set of data for said patient or device.

3. Data management according to claim 2, wherein the processing unit is adapted to generate a display control signal to display an ok-symbol for each person or device for whom or which no warning state or alarm state is given.

4. Data management system according to one of claims 1 to 3, wherein the processing unit is adapted to receive a user identification signal via the user interface and to immediately generated a display control signal for immediately displaying all actual alarm symbols given by an actual set of data being assigned to patients or devices assigned to the user identified by the identification signal.

5. Data management system according to claim 2 and 4, wherein the processing unit is adapted to generate a display control signal immediately after receiving a user identification signal said display control signal being adapted to display all actual warning states, which are previously selected and which are given for the patients or devices being assigned to a user, which is identified by the user identification signal.

6. Data management system according to one of claims 1 to 5, wherein the processing unit is adapted to generate a display control signal to display a list of all names of all patients or devices assigned to an identified user and to display for each patient or device an alarm symbol or a warning symbol or an ok-symbol associated to said names.

7. Data management system according to claim 6, wherein the processing unit is adapted to generate the display control signal for generating a display such that all names of patients or devices related to an actual alarm state are listed atop together with an alarm symbol.

8. Data management system according to claim 6, wherein the data interface comprises a select interface, which is connected to the processing unit and wherein the processing unit is adapted to retrieve further information assigned to a patient or a device being selected by a select signal received via the select interface and to generate a display control signal to display that further information.

9. Data management system according to claim 8, wherein said further information to be displayed for a selected patient or device comprises information to a set of categories characterizing a general status of the patient or the device.

10. Data management system according to claim 9, wherein the processing unit is adapted to allow a selection of each of the categories describing a general status of a patient or device and to initiate a display of all actual data being stored for said selected category.

11. A data storage means comprising a program for performing a graphical display of physiological or technical data or both, the program comprising the steps of:
retrieval of data from a data base
processing of the data
generating a display control signal dependent on said processing of the data

12. Data storage means according to claim 11, where a said step of processing said retrieved data comprises the sub steps of
accessing a rule base
applying rules stored in the rule base on said retrieved data determining alarm states defined by said rules and
generating a display control signal displaying an alarm symbol if an alarm state is determined.

13. Data management system according to claim 1, wherein the processing unit is connected to a communication unit which is adapted to generate a notification signal upon detection of a warning state or an alarm state as detected for a patient by the processing unit.

14. Data management system according to claim 13, wherein the data management system comprises a communication interface connected to the communication unit and being adapted to respond to the notification signal by transmitting of a notification message to a physician who is assigned to the patient showing selected information regarding the warning state or the alarm state via a selected configurable communication pathway, e.g., E-Mail, Fax, SMS or Pager.

15. Data management system according to claim 13 or 14, wherein the communication unit comprises a notification message configuration data base which can be individually configured by the physician using the user interface in a way that an individual notification profile can be created for each patient.

16. Data management system according to claim 15, wherein the notification message configuration data base comprises physician identification data elements, message trigger identification data elements and information selection data elements which each are assigned to an individual patient and to which each a value representing a communication pathway, a message triggering situation and information to be transmitted, respectively, can be assigned in groups or individually in order to represent notification profiles or configuration settings which can be modified, stored, and applied to other patients as well.
